# EUROPEAN PATENT SPECIFICATION

(11) **EP 2 391 398 B1**
(45) Date of publication and mention of the grant of the patent: **09.04.2014**
(21) Application number: 10705464.5
(22) Date of filing: 02.02.2010
(51) Int. Cl.: A61L 27/34, A61L 27/54, A61L 27/56, A61L 27/58

(54) **ANTIMICROBIAL ACCESSORY FOR AN IMPLANTABLE MEDICAL DEVICE**
ANTIMIKROBIELLES ZUBEHÖR FÜR EIN IMPLANTIERBARES MEDIZINPRODUKT
ACCESSOIRE ANTIMICROBIEN POUR DISPOSITIF MÉDICAL IMPLANTABLE

(30) Priority: 13.02.2009 US 152467 P; 30.10.2009 US 256758 P; 02.02.2009 US 149214 P; 18.06.2009 US 218328 P; 31.03.2009 US 165273 P
(43) Date of publication of application: 07.12.2011
(73) Proprietor: Medtronic, Inc., Minneapolis, MN 55342-5604 (US)
(72) Inventor: COBIAN, Kenneth, E., St. Anthony, MN 55418 (US); EICHENBERG GALLAGHER, Genevieve, Louise, Mendota Heights, MN 55120 (US); SEILER, Peter, M., St. Anthony Village, MN 55418 (US); DANG, Kiem, H., Blaine, MN 55449 (US); HEMENWAY, Michael, S., Mounds View, MN 55112 (US); YANG, Zhongping, Woodbury, MN 55129 (US); SCHULD, James, L., Plymouth, MN 55442 (US); BLUMENTHAL, Tico, Minneapolis, MN 55403 (US)
(74) Representative: Hughes, Andrea Michelle
(86) International application number: PCT/US2010/022944
(87) International publication number: WO 2010/088698

(56) References cited:
- WO-A1-2004/026361
- WO-A2-2008/039917
- WO-A2-2008/131089
- US-A- 5 217 493
- US-A1- 2004 186 528
- Edward M. Petrie: "Pressure sensitive adhesive for Health Care"[Online] 2 June 2004 (2004-06-02), XP002608676 SpecialChem Retrieved from the Internet: URL:www.specialchem4adhesives.com/resource s/print.aspx?id=774> [retrieved on 2010-11-08]
- Anonymous: "TyRx Pharma, Inc. Announces Food and Drug Administration (FDA) 510(k) Clearance of the AIGIS (Rx) (TM) Cardiac Rhythm Medical Device (CRMD) Anti-bacterial Envelope, an Innovative Mesh Envelope Designed to Immobilize and Reduce Bacterial Infection of a Pacemaker or Implantable Cardioverter Defribilla"[Online] 1 January 2008 (2008-01-01), XP002608677 BNET's Business Publications Retrieved from the Internet: URL:http://findarticles.com/p/articles/mi_ pwwi/is_20081/ai_n211947433/> [retrieved on 2010-11-06]

## Description

### TECHNICAL FIELD

The disclosure relates to implantable medical devices and, more particularly, to methods of reducing risk of post-implantation infection.

### BACKGROUND

Implantable medical devices (IMDs) include a variety of devices that provide therapy (such as electrical simulation or drug delivery) to a patient, monitor a physiological parameter of a patient, or both. IMDs typically include a number of functional components encased in a housing. The housing is implanted in a body of the patient. For example, the housing may be implanted in a pocket created in a torso of a patient. The housing may be constructed of a biocompatible material, such as titanium. While the housing is biocompatible, there may still be a risk of infection to the patient as a result of the implantation procedure or the presence of the IMD in the body.

WO2208039917 discloses medical devices for preventing post-implantation infection comprising an antimicrobials containing polymer coating layer. The antimicrobials can be disposed within one or more portions of the same polymeric coating layer or on different layers.

### SUMMARY

In general, the disclosure is directed to an antimicrobial accessory for an implantable medical device (IMD) and techniques for manufacturing the antimicrobial accessory.

In one aspect, the invention provides a system as defined in claim 1. The first antimicrobial may be different from the second antimicrobial. In some examples, the at least one domain comprising the first polymer and/or the at least one domain comprising the second polymer may additionally include an additive, such as an antioxidant or another excipient. The additive may be the same or different in the respective types of domains. In some examples, the first polymer, the second polymer, and/or the additive may be selected to affect an elution rate of the
antimicrobial in the respective domains. Such a patterned antimicrobial accessory may allow independent control of the elution rates of the first antimicrobial and the second antimicrobial. In some examples, the elution rates of the first and second antimicrobials may be controlled to be approximately equal. In other examples, the elution rates of the first and second antimicrobials may be controlled to be different than one another.

In one aspect, the disclosure is directed to an antimicrobial accessory comprising a layer including a pressure sensitive adhesive and an antimicrobial mixed substantially throughout the pressure sensitive adhesive. According to this aspect of the disclosure, the antimicrobial accessory is configured to be adhered to a housing of an implantable medical device.

In another aspect, the disclosure is directed to a system including an antimicrobial accessory comprising a layer including a pressure sensitive adhesive and an antimicrobial mixed substantially throughout the pressure sensitive adhesive. According to this aspect of the disclosure, the system further includes an implantable medical device comprising a housing, and the antimicrobial accessory is adhered to the housing by the pressure sensitive adhesive.

In a further aspect, the disclosure is directed to a method including forming a mixture comprising a pressure sensitive adhesive, a solvent, and an antimicrobial, forming the mixture into a layer, and removing the solvent from the mixture to form an antimicrobial accessory comprising the pressure sensitive adhesive and the antimicrobial.

In an additional aspect, the disclosure is directed to a system comprising an implantable medical device including a housing and an antimicrobial accessory. According to this aspect of the disclosure, the antimicrobial accessory includes at least one first domain comprising a pressure sensitive adhesive and a first antimicrobial mixed in the pressure sensitive adhesive, and at least one second domain comprising a second polymer and a second antimicrobial mixed in the second polymer. Further, the antimicrobial accessory is adhered to the housing by the at least one first domain.

In a further aspect, the disclosure is directed to a method including depositing minocycline in a first solvent selected from the group consisting of methanol, ethanol, and combinations thereof, depositing rifampin in a second solvent selected from the group consisting of ethyl acetate, tetrahydrofuran, and combinations thereof, and combining the first solvent including the first antimicrobial, the second solvent including the second antimicrobial, and a polymer into a substantially homogeneous mixture. According to this aspect of the disclosure, the method further includes forming the substantially homogeneous mixture into a shape and drying the shape to remove substantially all of the first solvent and the second solvent and form an antimicrobial accessory including the polymer, the first antimicrobial, and the second antimicrobial.

In another aspect, the disclosure is directed to a method including determining an implantable medical device is a candidate for an antimicrobial accessory to be adhered thereto, adhering the antimicrobial accessory to the implantable medical device, and implanting the implantable medical device with the antimicrobial accessory adhered thereto in a body of a patient.

### BRIEF DESCRIPTION OF DRAWINGS

FIG. 1 is a conceptual diagram illustrating an example therapy system that may be used to provide cardiac stimulation therapy to a patient.
FIG 2 is a cross-sectional diagram illustrating an example of an antimicrobial accessory including an adhesive layer attached to a housing of an implantable medical device.
FIG. 3 is a cross-sectional diagram illustrating an example of an antimicrobial accessory including a pressure sensitive adhesive attached to a housing of an implantable medical device.
FIG 4 is a conceptual diagram illustrating an example of an antimicrobial accessory including a sleeve attached to a housing of an implantable medical device.
FIG 5 is a cross-sectional diagram illustrating an example of an antimicrobial accessory including a pouch at least partially encapsulating a housing of an implantable medical device.
FIGS. 6A and 6B are flow diagrams illustrating example techniques of forming an antimicrobial accessory from an uncured polymer.
FIG. 7 is a flow diagram illustrating an example of a technique for forming an antimicrobial accessory including a pressure sensitive adhesive.
FIGS. 8A and 8B are flow diagrams illustrating example techniques of forming an antimicrobial accessory from a polymer dissolved in a solvent.
FIG. 9 is a flow diagram illustrating an example technique of milling an enhanced tear resistance silicone and an antimicrobial to form an antimicrobial accessory.
FIG 10 is a flow diagram illustrating an example technique of implanting an implantable medical device.
FIG. 11 is a conceptual diagram of an example of a patterned antimicrobial accessory.
FIG. 12 is a conceptual diagram of an example of a patterned antimicrobial accessory.
FIG. 13 is a conceptual diagram of an example of a patterned antimicrobial accessory.
FIG. 14 is a flow diagram illustrating an example of a technique for forming a patterned antimicrobial accessory.
FIG. 15 is a diagram of positions at which pressure sensitive adhesives were applied to a pacemaker housing for performing adhesion tests.
FIG. 16 is a diagram of positions at which pressure sensitive adhesives were initially applied to a pacemaker housing and positions to which the pressure sensitive adhesives were repositioned for performing adhesion tests.
FIG. 17 is a bar diagram illustrating a percent recovery of minocycline HCl and rifampin from examples of antimicrobial accessories of different compositions.
FIG. 18 is a line diagram illustrating an elution profile of minocycline HCl and rifampin from an example of an antimicrobial accessory.
FIG. 19 is a line diagram illustrating an elution profile of minocycline HCl and rifampin from an example of an antimicrobial accessory.
FIG. 20 is a line diagram illustrating an elution profile of minocycline HCl and rifampin from an example of an antimicrobial accessory.
FIG. 21 is a bar diagram illustrating zone of inhibition data collected for four examples of antimicrobial accessories.
FIG. 22 is a line diagram illustrating measurement of a percent of minocycline HCl released as a function of time from an example of an antimicrobial accessory.
FIG. 23 is a line diagram illustrating measurement of a percent of rifampin released as a function of time from an example of an antimicrobial accessory.
FIG. 24 is a line diagram illustrating in-vivo measurement of cumulative elution of rifampin and minocycline HCl from examples of antimicrobial accessories having different compositions.
FIG. 25 is a line diagram illustrating in-vitro measurement of cumulative elution of rifampin and minocycline HCl from examples of antimicrobial accessories having different compositions.

### DETAILED DESCRIPTION

In general, the disclosure is directed to an antimicrobial accessory for an implantable medical device (IMD).

The antimicrobial may include, for example, an antibiotic such as a tetracycline (e.g., minocycline, doxycycline), a rifamycin (e.g., rifampin, rifaximin, rifapentine, rifabutin), a macrolide (e.g., erythromycin), a penicillin (e.g., nafcillin), a cephalosporin (e.g., cefazolin), another beta-lactam antibiotic (e.g., imipenem, aztreonam) an aminoglycoside (e.g., gentamicin), a glycopeptide (e.g., vancomycin, teicoplanin), a quinolone (e.g., ciprofloxacin), fusidic acid, trimethoprim, metronidazole, mupirocin, a polene (e.g., amphotericin B), an azole (e.g., fluconazole) and a beta-lactam inhibitor (e.g., sulbactam), tigecycline, daptomycin, clindamycin, or another fluoroquinolone, bacitracin, neomycin, an antiseptic, an antimicrobial peptide, a quaternary ammonium, or the like. In some examples, the antimicrobial may be provided in a salt form, e.g., minocycline HCl, gentamicin crobefate, or genatamicin sulfate. The antimicrobial may be selected to provide efficacious prevention or treatment of any infection that may be present proximate to the implant site at which the IMD is implanted. In some examples, the antimicrobial accessory may include at least two antimicrobials, and the combination of the at least two antimicrobials may be selected to efficaciously treat or prevent any infection present proximate to the implant site of the IMD. In some examples, the antimicrobial accessory comprises minocycline and rifampin. In other examples, the antimicrobial comprises gentamicin, alone or in combination with another antimicrobial.

The antimicrobial accessory also includes a biocompatible polymer, and the antimicrobial may be mixed into the biocompatible polymer. In some examples, the biocompatible polymer is biodegradable, such that the antimicrobial accessory breaks down over time after being implanted in the patient. This may facilitate release of substantially all of the antimicrobial, which may reduce the risk of bacteria developing resistance to the antimicrobial in the antimicrobial accessory. A biodegradable antimicrobial accessory may also mitigate or prevent growth of bacteria on the antimicrobial accessory after the antimicrobial has eluted from the accessory. For example, the biodegradable polymer may break down over time after being implanted in the patient. In some examples, the biodegradable polymer may comprise collagen, poly(lactic-*co*-glycolic acid) (PLGA), poly(lactic acid) (PLA), poly(glycolic acid) (PGA), poly(ethylene oxide) (PEO), poly(ortho ester) (POE), poly(ε-caprolactone) (PCL), poly(dioxanone), polyglyconate, hyaluronic acid, gelatin, fibrin, fibrinogen, cellulose, starch, cellulose acetate, polyvinylpyrrolidone (PVP), a poly(ethylene oxide)/poly(propylene oxide) copolymer (PEO-PPO), poly(ethylene vinyl acetate), poly(hydroxybutyrate-covalerate), polyanhydride, poly(glycolic acid-*co*-trimethylene carbonate), polyphosphoester, polyphosphoester urethane, a poly(amino acid), a cyanoacrylate, poly(trimcthylene carbonate), poly(iminocarbonate), a copoly(ether-ester) such as PEO/PLA, a polyalkylene oxalate, a polyphasphazene, a polyarylate, a tyrosine-based biodegradable or bioabsorbable polymer, poly hydroxyalkanoate (PHA), a sugar ester, or the like..

In other examples, the biocompatible polymer is not biodegradable and may remain attached to the IMD indefinitely after the IMD has been implanted in the patient. For example, the polymer may include silicone or polyurethane. The silicone may include a silicone pressure sensitive adhesive (PSA), a room temperature vulcanization (curing) (RTV) silicone, an enhanced tear resistance (ETR) silicone, a liquid silicone rubber (LSR), or the like. For example, an RTV silicone, an ETR silicone, or a LSR may be produced by reacting a first constituent and a second constituent to form the RTV silicone, the ETR silicone, or LSR. In some examples, the antimicrobial may be mixed into the first constituent of the silicone prior to mixing the second constituent into the first constituent. In other examples, the antimicrobial may be mixed into the first and second constituents of the silicone after the first and second constituents have been mixed together. The first and second constituents may then react or cure to produce the cured silicone and be formed to the desired form factor of the antimicrobial accessory. In some examples in which two antimicrobials are mixed in the silicone, a first antimicrobial may be mixed in the first constituent and a second antimicrobial may be mixed in the second constituent. The first and second constituents, including the first and second antimicrobials, respectively, may then be mixed and cured to form the cured silicone including mixed therein the first and second antimicrobials.

In other examples, the silicone may include an RTV silicone that is formed from one component that cures at room temperature to form the cured silicone. In examples such as these, at least one antimicrobial may be mixed into the uncured RTV silicone constituent to form a substantially homogeneous mixture. The mixture may then be cured by exposure to atmospheric moisture, e.g., water vapor. Some such RTV silicone systems may include acetoxy, methoxy or ethoxy functional groups that react with water vapor and liberate acetic acid, methanol, or ethanol, respectively, during the curing process. As the RTV silicone cures, the mixture may be formed to the desired form factor of the antimicrobial accessory.

In some examples, the silicone may include an ETR silicone, which may be processed by milling to mix the first and second constituents of the silicone and the antimicrobial. Following mixing of the ETR silicone and the antimicrobial, the ETR silicone and the antimicrobial may be cured to form the cured ETR silicone and formed into a desired form factor for the antimicrobial accessory. For example, the ETR silicone and the antimicrobial may be extruded or molded to begin the reaction of the first and second constituents and the cure of the ETR silicone, and may be formed into a desired form factor, e.g., a sheet or film, by the extrusion or molding process.

In other examples, the antimicrobial accessory includes a PSA. The PSA may include a silicone PSA, or may include, for example, an acrylic PSA, a polyisobutylene PSA, a polyurethane PSA, a cyanoacrylate PSA, a PLGA-based PSA, or the like. In some examples, the PSA may be delivered in a solvent. For example, a silicone PSA may be delivered as 60 weight percent (wt. %) solids (i.e., silicone) in ethyl acetate. A first of at least two antimicrobials may be mixed the in solvent carrying the PSA, while a second of the at least two antimicrobials may be mixed in another solvent. The two mixtures may then be mixed together and dried to remove the solvent and form the antimicrobial accessory. Because PSAs may have little or no cross-linking, an antimicrobial accessory including a PSA may release the drug at a higher elution rate than an antimicrobial accessory including an antimicrobial mixed in a more cross-linked polymer (i.e., a polymer having a higher crosslink density). This may be advantageous because it facilitates a higher initial dosage of the antimicrobial to the implant site.

The antimicrobial accessory may be formed into one of a variety of form factors, including, for example, a disk, a sheet, or a film. When the antimicrobial accessory includes a disk, sheet, or film, the antimicrobial accessory may include a pressure sensitive adhesive into which the antimicrobial is mixed, or may include an adhesive layer applied to at least one side of a polymer layer into which the antimicrobial is mixed. The antimicrobial accessory then is adhered to a housing of the IMD. In examples in which the polymer is a PSA, the antimicrobial accessory may not include a separate PSA layer.

In other examples, the antimicrobial accessory may be formed into a sleeve. The sleeve may be sized and configured to fit over a housing of the IMD. The antimicrobial sleeve may include a polymer and at least one antimicrobial. The sleeve is adhered to the housing by an adhesive,

The antimicrobial accessory may be formed into a pouch that at least partially encloses a housing of an IMD. The pouch may be adhered to the housing by an adhesive applied to a surface of the pouch. The pouch may define one or more aperture that permits any leads or catheters connected to the IMD to extend out of the pouch. In other examples, the pouch may enclose only portion of the housing, and may not enclose the portion of the housing from which a lead or catheter extends.

The antimicrobial accessory comprises a patterned antimicrobial, including at least one first domain that includes a first polymer and a first antimicrobial and at least one second domain that includes a second polymer and a second antimicrobial. The first polymer is a pressure sensitive adhesive. The second polymer may be different than the first polymer. For examples, the second polymer may comprise silicone, polyurethane, or one of the biodegradable polymers described herein. The first and second antimicrobials may be the same or may be different. In some examples, the combinations of the first polymer and the first antimicrobial and the second polymer and the second antimicrobial, respectively, may be made based on considerations including chemical compatibility between the polymer and the antimicrobial, elution rate of the antimicrobial from the polymer, manufacturing issues (e.g., curing temperatures of the polymer and temperature stability of the antimicrobials), or the like. The first domain and the second domain comprise different compositions.

Additionally and optionally, at least one of the first domain and the second domain may include an additive mixed into the first polymer or the second polymer, respectively. The additive may be, for example, an antioxidant, plasticizer or another excipient. An antioxidant may reduce oxidation, and thus improve shelf-life, of the antimicrobial in the domain in which the antioxidant is mixed. A plasticizer or excipient may affect an elution rate of the antimicrobial in the domain in which the plasticizer or excipient is mixed.

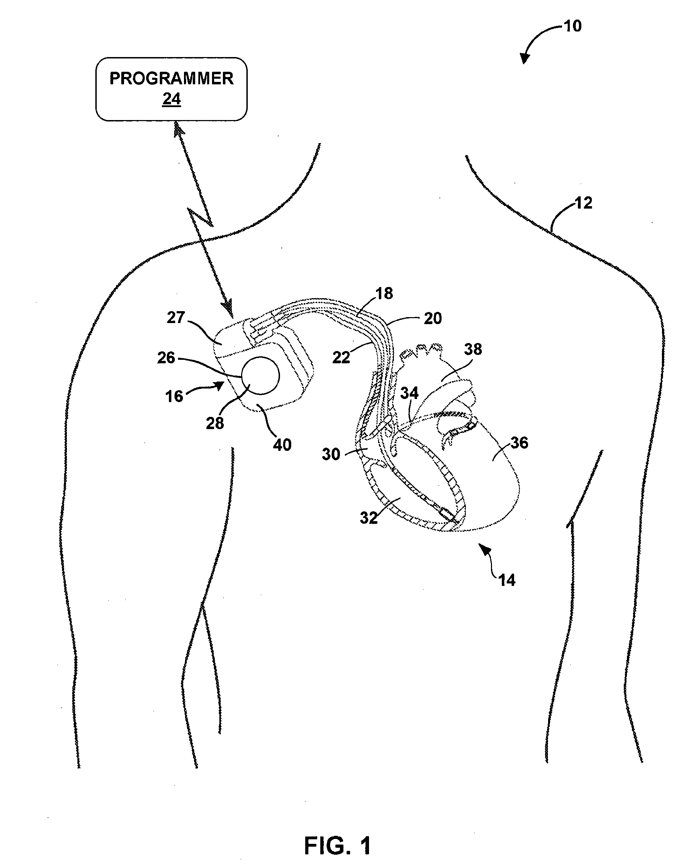

FIG. 1 is a conceptual diagram illustrating an example therapy system 10 that may be used to provide therapy to a patient 12. Patient 12 ordinarily, but not necessarily, will be a human. Therapy system 10 may include an implantable cardiac device (ICD) 16, and a programmer 24. In the example illustrated in FIG. 1, ICD 16 has an antimicrobial accessory 26 adhered to a surface of a housing of ICD 16.

While the examples in the disclosure are primarily directed to an antimicrobial accessory 26 adhered to an ICD 16, in other examples, antimicrobial accessory 26 may be utilized with other implantable medical devices. For example, antimicrobial accessory 26 may be adhered to an implantable drug delivery device, an implantable monitoring device that monitors one or more physiological parameter of patient 12, an implantable neurostimulator (e.g., a spinal cord stimulator, a deep brain stimulator, a pelvic floor stimulator, a peripheral nerve stimulator, or the like), a cardiac or neurological lead, a catheter, an orthopedic device such as a spinal device, or the like. In general, antimicrobial accessory 26 is adhered to any medical device configured to be implanted in a body of a patient 12.

In the example depicted in FIG. 1, ICD 16 is connected (or "coupled") to leads 18, 20, and 22. ICD 16 may be, for example, a device that provides cardiac rhythm management therapy to heart 14, and may include, for example, an implantable pacemaker, cardioverter, and/or defibrillator that provides therapy to heart 14 of patient 12 via electrodes coupled to one or more of leads 18, 20, and 22. In some examples, ICD 16 may deliver pacing pulses, but not cardioversion or defibrillation shocks, while in other examples, ICD 16 may deliver cardioversion or defibrillation shocks, but not pacing pulses. In addition, in further examples, ICD 16 may deliver pacing pulses, cardioversion shocks, and defibrillation shocks.

Leads 18, 20, 22 that are coupled to ICD 16 extend into the heart 14 of patient 12 to sense electrical activity of heart 14 and/or deliver electrical stimulation to heart 14. In the example shown in FIG. 1, right ventricular (RV) lead 18 extends through one or more veins (not shown), the superior vena cava (not shown), and right atrium 30, and into right ventricle 32. Left ventricular (LV) coronary sinus lead 20 extends through one or more veins, the vena cava, right atrium 30, and into the coronary sinus 34 to a region adjacent to the free wall of left ventricle 36 of heart 14. Right atrial (RA) lead 22 extends through one or more veins and the vena cava, and into the right atrium 30 of heart 14. In other examples, ICD 16 may deliver stimulation therapy to heart 14 by delivering stimulation to an extravascular tissue site in addition to or instead of delivering stimulation via electrodes of intravascular leads 18, 20, 22.

ICD 16 may sense electrical signals attendant to the depolarization and repolarization of heart 14 (e.g., cardiac signals) via electrodes (not shown in FIG. 1) coupled to at least one of the leads 18, 20, 22. In some examples, ICD 16 provides pacing pulses to heart 14 based on the cardiac signals sensed within heart 14. The configurations of electrodes used by ICD 16 for sensing and pacing may be unipolar or bipolar. ICD 16 may also provide defibrillation therapy and/or cardioversion therapy via electrodes located on at least one of the leads 18, 20, 22. ICD 16 may detect arrhythmia of heart 14, such as fibrillation of ventricles 32 and 36, and deliver defibrillation therapy to heart 14 in the form of electrical shocks. In some examples, ICD 16 may be programmed to deliver a progression of therapies, e.g., shocks with increasing energy levels, until a fibrillation of heart 14 is stopped. ICD 16 may detect fibrillation by employing one or more fibrillation detection techniques known in the art. For example, ICD 16 may identify cardiac parameters of the cardiac signal, e.g., R-waves, and detect fibrillation based on the identified cardiac parameters.

In some examples, programmer 24 may be a handheld computing device or a computer workstation. Programmer 24 may include a user interface that receives input from a user. The user interface may include, for example, a keypad and a display, which may be, for example, a cathode ray tube (CRT) display, a liquid crystal display (LCD) or light emitting diode (LED) display. The keypad may take the form of an alphanumeric keypad or a reduced set of keys associated with particular functions. Programmer 24 can additionally or alternatively include a peripheral pointing device, such as a mouse, via which a user may interact with the user interface. In some embodiments, a display of programmer 24 may include a touch screen display, and a user may interact with programmer 24 via the display.

A user, such as a physician, technician, or other clinician, may interact with programmer 24 to communicate with ICD 16. For example, the user may interact with programmer 24 to retrieve physiological or diagnostic information from ICD 16. A user may also interact with programmer 24 to program ICD 16, e.g., select values for operational parameters of ICD 16.

Programmer 24 may communicate with ICD 16 via wireless communication using any techniques known in the art. Examples of communication techniques may include, for example, low frequency or radiofrequency (RF) telemetry, but other techniques are also contemplated. In some examples, programmer 24 may include a programming head that may be placed proximate to the patient's body near the ICD 16 implant site in order to improve the quality or security of communication between ICD 16 and programmer 24.

The antimicrobial is mixed into the polymer. As described above, antimicrobial accessory 26 may reduce or substantially eliminate risk of post-implant infection proximate to the implant site of ICD 16 by releasing the antimicrobial over a period of time subsequent to implantation of ICD 16 in the body of patient 12.

The at least one antimicrobial may include, for example, an antibiotic such as a tetracycline (e.g., minocycline, doxycycline), a rifamycin (e.g., rifampin, rifaximin, rifapentine, rifabutin), a macrolide (e.g., erythromycin), a penicillin (e.g., nafcillin), a cephalosporin (e.g., cefazolin), another beta-lactam antibiotic (e.g., imipenem, aztreonam) an aminoglycoside (e.g., gentamicin), a glycopeptide (e.g., vancomycin, teicoplanin), a quinolone (e.g., ciprofloxacin), fusidic acid, trimethoprim, metronidazole, mupirocin, a polene (e.g., amphotericin B), an azole (e.g., fluconazole) and a beta-lactam inhibitor (e.g., sulbactam), tigecycline, daptomycin, clindamycin, or another fluoroquinolone, bacitracin, neomycin, an antiseptic, an antimicrobial peptide, a quaternary ammonium, or the like. In some examples, the antimicrobial may be provided in a salt form, e.g., minocycline HCl, gentamicin crobefate, or gentamicin sulfate. In some examples, two or more antimicrobials may be selected to efficaciously prevent or treat any infection present proximate to the implant location of ICD 16, e.g., infection in the pocket in which ICD 16 is implanted. For example, one combination of antimicrobials that may be utilized is minocycline and rifampin.

Antimicrobial accessory 26 may include a biocompatible polymer. The antimicrobial may be mixed into the biocompatible polymer. In some examples, the biocompatible polymer may be biodegradable or bioabsorbable and be absorbed by the body of patient 12 after implantation of antimicrobial accessory 26. In other embodiments, antimicrobial accessory 26 is not biodegradable and may remain in the body of patient 12 after implantation.

The biocompatible polymer may include, for example, a polyurethane or a silicone. Various types of silicone may be used, including, for example, silicone pressure sensitive adhesive (PSA), room temperature vulcanization (curing) (RTV) silicone, liquid silicone rubber (LSR), enhanced tear resistance (ETR) silicone, or the like. Exemplary silicones include, but are not limited to, Silastic® Q-7-4850 LSR, available from Dow Coming, Corp., Midland, MI; Silastic® MDX4-4210, available from Dow Coming, Corp., Midland, MI; Q7-4735, Q7-4750, and Q7-4765 ETR silicones, available from Dow Corning, Corp., Midland, MI; NuSil MED-1137 and NuSil MED-200 RTV silicones, available from NuSil Technology, LLC, Carpinteria, CA; Rehau SI-1511 RTV silicone, available from Rehau Co., Leesburg, VA; Silastic® MDX7-4502, BIO-PSA 7-4501, BIO-PSA 7-4402, BIO-PSA 7-4502, BIO-PSA-4602, 7-9800 SSA, and MG7-9850 PSA silicones, available from Dow Coming, Corp., Midland, MI. In some examples, the at least one antimicrobial may be mixed into the silicone or a constituent of the silicone, prior to curing, while in other embodiments, the at least one antimicrobial may be mixed into the silicone subsequent to curing of the silicone. Further details regarding the processing and manufacture of a silicone antimicrobial accessory will be described below.

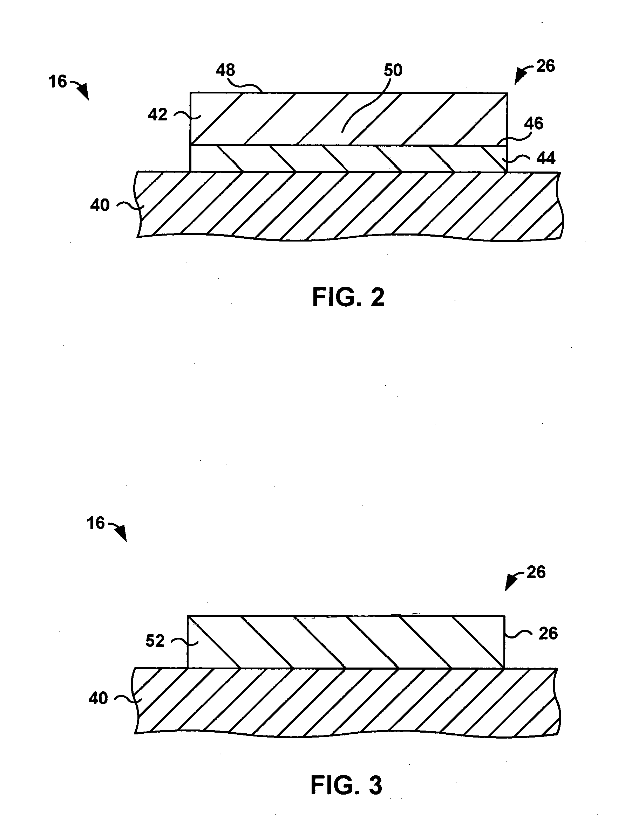

In other examples, the biocompatible polymer may include another PSA, such as an acrylic PSA, a polyisobutylene PSA, a polyurethane PSA, a cyanoacrylate PSA, a PLGA-based PSA, or the like. Antimicrobial accessory 26 may be formed of a single layer when formed from a PSA, as will be described in further detail with reference to FIG. 3. In examples such as these, the at least one antimicrobial may be mixed into the PSA. Forming antimicrobial accessory 26 from a PSA may result in a higher release rate (elution rate) of the antimicrobial than from a polymer with a higher cross-link density, because the PSA has little or no cross-linking.

The biocompatible polymer may also include a biodegradable or bioabsorbable polymer, such as, for example, collagen, PLGA, PLA, PGA, PEO, POE, PCL, poly(dioxanone), polyglyconate, hyaluronic acid, gelatin, fibrin, fibrinogen, cellulose, starch, cellulose acetate, PVP, a PEO/PPO copolymer, poly(ethylene vinyl acetate), poly(hydroxybutyrate-covalerate), polyanhydride, poly(glycolic acid-co-trimethylene carbonate), polyphosphoester, polyphosphoester urethane, a poly(amino acid), a cyanoacrylate, poly(trimethylene carbonate), poly(iminocarbonate), a copoly(ether-ester) such as PEO/PLA, a polyalkylene oxalate, a polyphasphazene, a polyarylate, a tyrosine-based biodegradable or bioabsorbable polymer, PHA, a sugar ester, or the like. The biodegradable or bioabsorbable polymer may degrade and be absorbed by the body of patient 12 over time after implantation of antibiotic accessory 26 in the body of patient 12. This may be advantageous because it may ensure that substantially all the antimicrobial is released from antimicrobial accessory 26, which may reduce risk of the growth or development of organisms that are resistant to the antimicrobial. Further, absorption of antimicrobial accessory 26 over time may remove a site at which bacteria can grow.

Regardless of the particular polymer from which antimicrobial accessory 26 is formed, antimicrobial accessory 26 may include other components that may influence the properties of the accessory 26. For example, antimicrobial accessory 26 may include an additive that influences the release rate of the antimicrobial from antimicrobial accessory 26, such as a plasticizer or another excipient. A plasticizer or excipient may affect the viscosity of the polymer in antimicrobial accessory 26, which may in turn affect the release rate of the at least one antimicrobial. Thus, incorporation of a plasticizer or excipient may be one manner in which the time over which the antimicrobial is released from antimicrobial accessory 26 is affected. In some examples, the additive may swell or dissolve in biological fluids present in the body of patient 12, which may affect the release rate of the antimicrobial. Exemplary additives that influence the release rate of the antimicrobial accessory may include, for example, poly(acrylic acid), poly(methacrylic acid), poly(vinylpyrolidone), a sugar ester, macrogol 15 hydroxystearate (IV), poly(lactic acid), lactic acid, glycerol, poly(ethylene glycol) (PEG), methyl polyethylene glycol (methyl PEG), poly(glycolic acid), poly(ε-caprolactum), polysorbate 80 (polyoxyethylene (20) sorbitan monooleate), polysorbate 20 (polyoxyethylene (20) sorbitan monolaurate), salts such as KCl, cationic surfactants, anionic surfactants, natural surfactants, or the like. Exemplary surfactants include, but are not limited to, sodium dodecyl sulfate (SDS), sodium stearate, sucrose stearate, stearyl alcohol, glycerol monostearate, mannitol, sodium laureth sulfate, sodium lauryl sulfate, triton X 100, sorbitol, fructose, chitosan, hyaluronic acid, alginate, and trimethyldodecylammonium (TMDA). As another example, antimicrobial accessory 26 may include fumed silica. Fumed silica may increase polymer integrity, such as integrity of a silicone PSA, and may also facilitate faster release of the antimicrobial. In some examples, the additive that influences the release rate of the antimicrobial from antimicrobial accessory 26 may constitute less than approximately 1 weight percent (wt. %) of antimicrobial accessory 26.

In some examples, antimicrobial accessory 26 may also include an antioxidant, which may reduce or substantially prevent oxidation of the antimicrobial. Exemplary antioxidants include, but are not limited to, monofunctional hindered phenolic antioxidants, such as butylated hydroxyl toluene (BHT), vitamin E, vitamin A, vitamin C, or those available under the trade designation Ciba® Irganox® 1076 or Ciba® Irganox® 1010, from BASF, Florham Park, N.J. In some examples, antimicrobial accessory 26 may include between approximately 0.1 wt. % and approximately 2 wt. % antioxidant.

In the example illustrated in FIG. 1, antimicrobial accessory 26 is in the form of a disk 28. Disk 28 may be adhered to housing 40 by an adhesive. In some examples, as illustrated in FIG. 2, a layer of adhesive 44, such as, for example, a silicone PSA, an acrylic PSA, a polyurethane PSA, a cyanoacrylate PSA, a PLGA-based PSA, or polyisobutylene PSA, may be applied to a surface 46 of a polymer layer 42 which includes mixed therein at least one antimicrobial. The adhesive 44 may be applied to the surface 46 of polymer layer 42 by, for example, spray coating, knife coating, air knife coating, gap coating, gravure coating, slot die coating, metering rod coating, doctor blade, or the like.

As illustrated in FIG. 3, the antimicrobial is mixed in a PSA 52, which then both adheres disk 28 to housing 40 and carries the antimicrobial. In these examples, the PSA may include, for example, a silicone PSA, an acrylic PSA, a polyurethane PSA, a cyanoacrylate PSA, a PLGA-based PSA, or polyisobutylene PSA. Mixing the antimicrobial directly in a PSA 52 may be advantageous because it may facilitate a faster antimicrobial release rate than a cross-linked polymer. Additionally or alternatively, mixing the antimicrobial directly in PSA 52 may simplify manufacture of disk 28 compared to a disk 28 including a polymer layer and a separate adhesive 44, because mixing the antimicrobial directly in PSA 52 results in a disk 28 having a single layer, instead of a multilayer disk 28.

Regardless of whether the adhesive is a separate layer of disk 28 or includes the antimicrobial mixed therein, disk 28 may be disposed on a release liner, such as a fluoropolymer release liner, to provide a convenient article for storing, shipping, and providing antimicrobial accessory 26 to the implanting clinician. In some examples, disk 28 disposed on the release liner may be packaged in a foil package or other substantially air and water impermeable package that is vacuum sealed or backfilled with an inert gas. Disk 28 may then be sterilized by, for example, electron beam, gamma beam, ethylene oxide, autoclaving, or the like.

Disk 28 may include a range of thicknesses, such as, for example, from about 0.0005 inch to about 0.100 inch (0.0127 mm - 2.54 cm). The thickness of disk 28 may affect the release rate of antimicrobial from antimicrobial accessory 26, particularly as the volume of antimicrobial accessory adjacent to surface 48 is depleted of antimicrobial and the antimicrobial must diffuse from an inner volume 50 of antimicrobial accessory 26 to surface 48 to be released into the body. In addition, the thickness of antimicrobial accessory 26, along with the diameter of the accessory 26, may affect the total amount of antimicrobial which is carried by the antimicrobial accessory 26. In some examples, disk 28 may have a diameter between approximately 0.125 inches (3.175 mm) and approximately 4 inches (10.16 cm).

Antimicrobial accessory 26 may be formed by a variety of techniques. For example, disk 28 may comprise a silicone or another polymer formed from two or more constituent parts. The at least one antimicrobial may be mixed in a first constituent of the silicone, and the second constituent may be added to the first constituent to initiate reaction of the constituents to form the silicone. In other examples, the first and second constituents may be mixed with each other, and then the at least one antimicrobial may be mixed into the mixture of the firs and second constituents. The first constituent and the at least one antimicrobial, and the second constituent and the first constituent, may be mixed using a variety of mixers, including, for example, a single-screw or twin-screw extruder, a Brabender mixer, a static mixer, an adhesive dispenser, or the like. The mixture of the two constituents and the at least one antimicrobial may then be formed to a desired shape, which may be, for example, the shape of antimicrobial accessory 26 (e.g., disk 28) or may be another shape, such as a sheet from which antimicrobial accessory 26 is later cut or otherwise formed. The mixture may be formed to the desired shape by injection molding, compression molding, transfer molding, casting, solvent dispersion followed by casting, spraying, extruding, painting, or the like. Finally, the mixture may be cured to allow the two constituents to react and form the silicone. In some examples, the uncured silicone including the antimicrobial may be deposited onto a release liner and passed through a furnace to effect or hasten cure of the silicone. The cured polymer and release liner may then be cut or stamped into the shape of antimicrobial accessory 26, packaged in a foil package, and sterilized, as described above.

In some examples, antimicrobial accessory 26 may be formed from two or more constituent parts and may include at least two antimicrobials. In some examples, a first antimicrobial may be mixed in a first constituent part and a second antimicrobial may be mixed in a second constituent part. The first and second constituent parts may then be mixed to react the constituents and formed the cured silicone with the first and second antimicrobials mixed therein.

In other examples, antimicrobial accessory 26 may include at least two antimicrobials, and the antimicrobials may dissolve more effectively in different solvents. Antimicrobial accessory 26 may be formed by dissolving a polymer and a first of the at least two antimicrobials in a first solvent, and dissolving a second of the at least two antimicrobials in a second solvent. The two solutions may then be mixed to produce a substantially homogeneous mixture including the polymer, the first and second antimicrobials, and the two solvents. The solvent may then be evaporated, leaving the dried polymer having the at least two antimicrobials mixed therein. In some examples, a first antimicrobial may be mixed in a first solvent, a second antimicrobial may be mixed in a second solvent, and the polymer may be mixed in a third solvent. The third solvent may be the same as the first solvent or the second solvent, or may be different from each of the first and second solvents.

For example, a PSA may be provided from a producer of the PSA as a solid carried in a first solvent, e.g., a silicone PSA carried in ethyl acetate. A first antimicrobial, such as rifampin, may be dissolved in the ethyl acetate, while a second antimicrobial, such as minocycline, may be dissolved in a second solvent, such as methanol or ethanol. The two mixtures may be mixed to form a single substantially homogeneous mixture, and then dried to remove substantially all of the solvent and form a film or other object. The film or other object may be in the form factor of antimicrobial accessory 26, or may be in another shape that is then further processed, e.g., cut or stamped to form antimicrobial accessory 26. In some examples, the PSA including the antimicrobial may be disposed on a release liner, and may be packaged in a foil package and sterilized, as described above.

As another example, an antimicrobial may be mixed in a biodegradable or bioabsorbable polymer using two or more solvents. For example, PLGA may be dissolved in tetrahydrofuran (THF). A first antimicrobial, such as rifampin may be dissolved in THF, either the THF in which PLGA is dissolved, or a separate volume of THF. A second antimicrobial, such as minocycline, may be dissolved in methanol or ethanol. The mixtures are then combined, dried, and formed to a desired shape to produce the antimicrobial accessory 26, as described above.

Although antimicrobial accessory 26 in the form of disk 28 has been described as being attached to housing 40 of ICD 16 by an adhesive, e.g., adhesive 44, in other examples (outside the scope of the claims), antimicrobial accessory 26 may be attached to ICD16 by other means, such as, for example, a suture or staple to connector block 27 of ICD 16 or an aperture defined in connector block 27. Connector block 27 may be formed of a polymer. In other examples (outside the scope of the claims), antimicrobial accessory 26 may not be attached to housing 40 in any manner, and may simply be implanted in patient 12 proximate to ICD 16 (e.g., at the implant site, next to ICD 16). These methods may be advantageous when antimicrobial accessory 26 includes a biodegradable polymer, because no adhesive residue will be left on a surface of housing 40. In some examples, the suture may also be biodegradable.

While FIG. 1 illustrates a disk 28, in other examples, antimicrobial accessory 26 may include a different form factor. For example, antimicrobial accessory 26 may include a sheet or film, which is adhered to housing 40. The sheet, film, or disk 28 may be applied to a single surface of housing 40, or may be applied to two or more surfaces of housing 40. The sheet or film may include a thickness similar to those described with respect to disk 28. Further the sheet or film may be manufactured by similar processes to disk 28, and may be packaged and sterilized similarly.

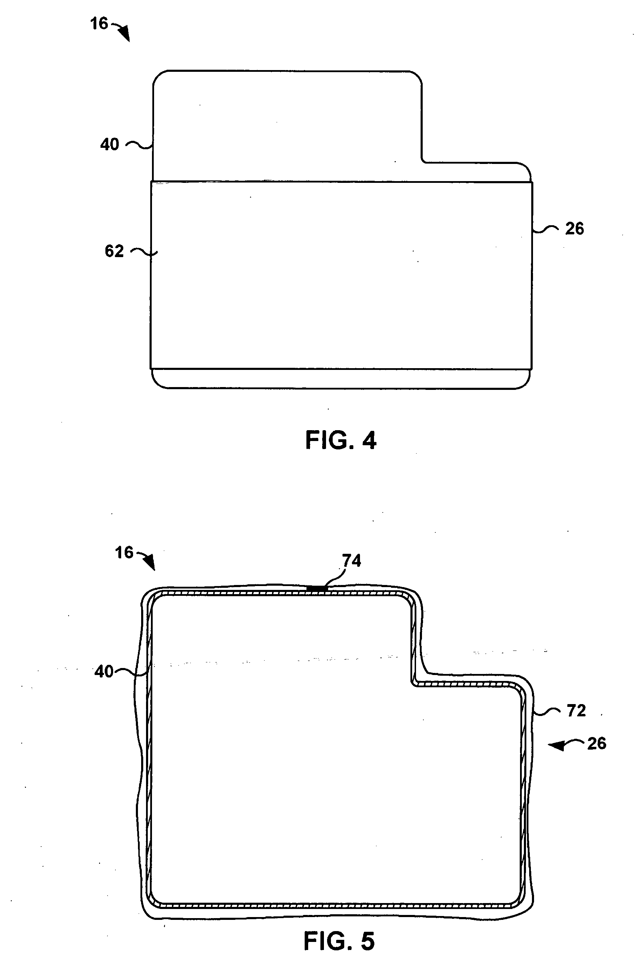

In other examples, as shown in FIG. 4, antimicrobial accessory 26 includes a sleeve 62. Sleeve 62 may be sized and configured to fit over housing 40 of ICD 16, as shown in FIG. 4. In some examples, sleeve 62 is sized such that a friction fit is formed between a surface of sleeve 62 and housing 40. In other examples, sleeve 62 may include a layer of adhesive applied to at least part of the surface that contacts housing 40, or may be formed of a PSA, as described above with respect to FIG. 3. The adhesive, whether applied to a surface of sleeve 62 or integrated into antimicrobial accessory 26, may adhere sleeve 62 to housing 40 and prevent migration of sleeve 62 relative to housing 40.

Similar to disk 28, sleeve may include a thickness of approximately 0.0005 inches (0.0127 mm) to approximately 0.10 inches (2.54 mm). The thickness may be selected based on, for example, the desired capacity of antimicrobial and/or the desired release time for the antimicrobial from sleeve 62. As described above, a thicker antimicrobial accessory 26 may increase the time required for substantially all of the antimicrobial to be released from the accessory 26. Conversely, a thinner antimicrobial accessory 26 may reduce the time required for substantially all of the antimicrobial to be released from the accessory 26.

FIG. 5 is a cross-sectional diagram that depicts another example of an antimicrobial accessory 26, which is a pouch 72 that at least partially encloses housing 40. In the example illustrated in FIG. 5, pouch 72 substantially fully encloses housing 40. As used herein substantially fully enclosing refers to a pouch 72 to a fully encloses the housing 40, but which may define at least one aperture that permits a lead, catheter, or other probe to extend from ICD 16 and out of pouch 72. In some examples, pouch 72 may be attached to housing 40 by an adhesive 74. Adhesive 74 may also function to close an opening in pouch 72 through which ICD 16 is inserted into pouch 72. In other examples, pouch 72 simply fits around housing 40. In some examples, the opening in pouch 72 through which ICD 16 is inserted may be closed by welding, melting, or adhering two portions of pouch 72 together to form a substantially continuous pouch 72.

Pouch 72 may include a range of thicknesses from about 0.00025 inches (0.00635 mm) to about 0.10 inches (2.54 mm). Pouch 72 may be sized and configured to fit intimately over housing 40, or may be sized and configured to fit more loosely over housing 40. In addition, pouch 72 may be customized for an individual ICD 16 or a type of ICD 16 or another IMD, or may be formed more generically and may fit over a wider range of IMDs.

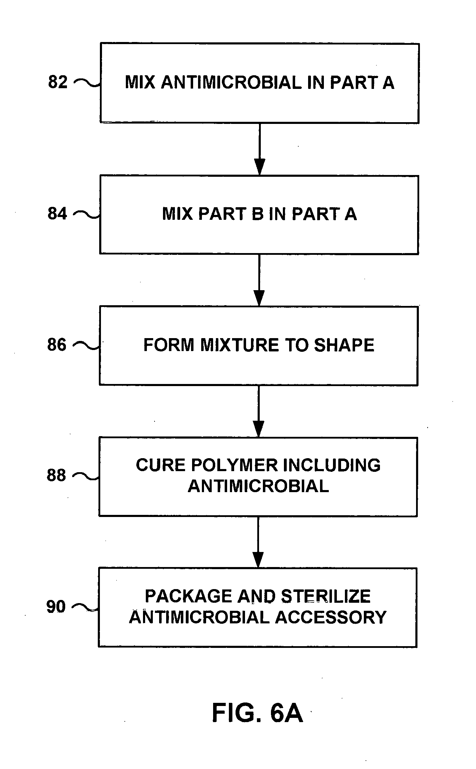

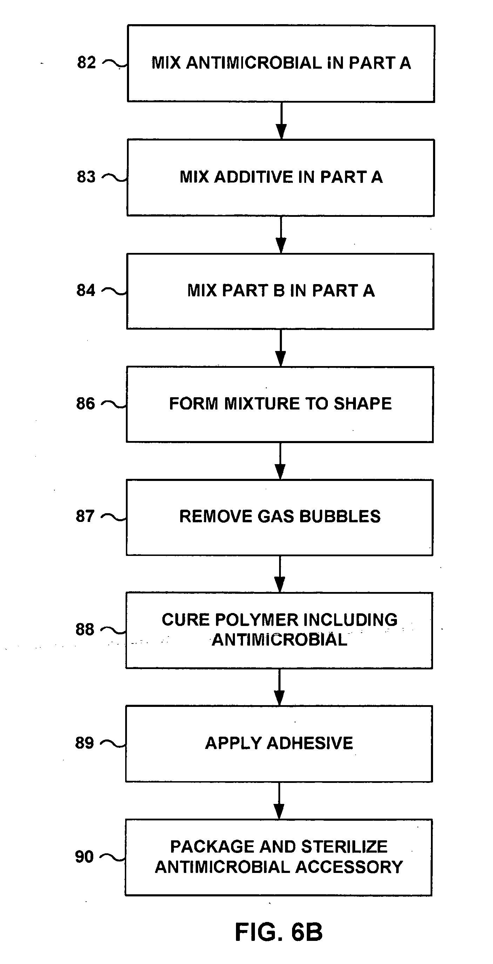

FIGS. 6A and 6B are flow diagrams of exemplary techniques of forming an antimicrobial accessory 26 by mixing the at least one antimicrobial in an uncured polymer and curing the polymer to form the accessory 26. Although the techniques of FIGS. 6A and 6B are similar in many respects, the technique described with reference to FIG. 6B includes additional steps that may be utilized in some examples. The techniques of FIGS. 6A and 6B will be described with respect to a silicone; however, the technique may also be utilized with another uncured polymer. The silicone described in FIGS. 6A and 6B may be, for example, an LSR, an RTV silicone, or the like.

In the technique illustrated in FIGS. 6A and 6B, the uncured silicone may be provided as two or more separate constituents, referred to herein as "part A" and "part B." To produce the actual (cured) silicone, parts A and B are mixed and allowed to cure. In some examples, parts A and B are mixed in the presence of a catalyst that facilitates the reaction between parts A and B. Certain parts A and B may react and cure to form the silicone at approximately room temperature, and the silicone may be referred to as an RTV silicone. In other examples, the parts A and B may require an elevated temperature to react and cure. In many examples, the time required for a substantially complete cure is at least partially dependent on the temperature at which the cure is effected. A balance between curing time and curing temperature may be selected to limit or prevent degradation of the antimicrobial due to elevated temperatures while accomplishing cure of the silicone in a reasonable length of time.

As shown in FIGS. 6A and 6B, at least one antimicrobial may be mixed in part A (82). The at least one antimicrobial may include, for example, an antibiotic such as a tetracycline (e.g., minocycline, doxycycline), a rifamycin (e.g., rifampin, rifaximin, rifapentine, rifabutin), a macrolide (e.g., erythromycin), a penicillin (e.g., nafcillin), a cephalosporin (e.g., cefazolin), another beta-lactam antibiotic (e.g., imipenem, aztreonam) an aminoglycoside (e.g., gentamicin), a glycopeptide (e.g., vancomycin, teicoplanin), a quinolone (e.g., ciprofloxacin), fusidic acid, trimethoprim, metronidazole, mupirocin, a polene (e.g., amphotericin B), an azole (e.g., fluconazole) and a beta-lactam inhibitor (e.g., sulbactam), tigecycline, daptomycin, clindamycin, or another fluoroquinolone, bacitracin, neomycin, an antiseptic, an antimicrobial peptide, a quaternary ammonium, or the like. In some examples, the antimicrobial may be provided in a salt form, e.g., minocycline HCl, gentamicin crobefate, or gentamicin sulfate. In some examples, a single antimicrobial is mixed in part A, while in other examples, at least two antimicrobials, such as minocycline and rifampin, are mixed in the part A. The mixing of the at least one antimicrobial and the part A may be accomplished using, for example, a single-screw extruder, a twin-screw extruder, a static mixer, a Brabender mixer, or the like. The at least one antimicrobial and the part A may be mixed to form a first substantially homogeneous mixture.

In some examples, as shown in FIG. 6B, an additive may be mixed into part A (83), along with the at least one antimicrobial. The additive may include an antioxidant, which may reduce or eliminate degradation of the at least one antimicrobial due to oxidation. Exemplary antioxidants include, but are not limited to, monofunctional hindered phenolic antioxidants, such as butylated hydroxyl toluene (BHT), vitamin E, vitamin A, vitamin C, or those available under the trade designation Ciba® Irganox® 1076 or Ciba® Irganox® 1010, from BASF, Florham Park, N.J. In some examples, antimicrobial accessory 26 may include between approximately 0.1 wt. % and approximately 2 wt. % antioxidant.

The additive may also be an additive that affects the release rate or elution rate of the at least one antimicrobial from antimicrobial accessory 26. For example, the additive that affects the release rate may include a plasticizer or another excipient. A plasticizer or excipient may affect the viscosity of the polymer in antimicrobial accessory 26, which may in turn affect the release rate of the at least one antimicrobial. Thus, incorporation of a plasticizer or excipient may be one manner in which the time over which the antimicrobial is released from antimicrobial accessory 26 is affected. In some examples, the additive may swell or dissolve in biological fluids present in the body of patient 12, which may affect the release rate of the antimicrobial. Exemplary additives that influence the release rate of the antimicrobial accessory may include, for example, poly(acrylic acid), poly(methacrylic acid), poly(vinylpyrolidone), a sugar ester, macrogol 15 hydroxystearate (IV), poly(lactic acid), lactic acid, glycerol, PEG, methyl PEG, poly(glycolic acid), poly(ε-caprolactum), polysorbate 80 (polyoxyethylene (20) sorbitan monooleate), polysorbate 20 (polyoxyethylene (20) sorbitan monolaurate), salts such as KCl, cationic surfactants, anionic surfactants, natural surfactants, or the like. Exemplary surfactants include, but are not limited to, SDS, sodium stearate, sucrose stearate, stearyl alcohol, glycerol monostearate, mannitol, sodium laureth sulfate, sodium lauryl sulfate, triton X 100, sorbitol, fructose, chitosan, hyaluronic acid, alginate, and TMDA. As another example, antimicrobial accessory 26 may include fumed silica. Fumed silica may increase polymer integrity and may facilitate faster release of the antimicrobial. In some examples, the additive that influences the release rate of the antimicrobial accessory may constitute less than approximately 1 weight percent (wt. %) of antimicrobial accessory 26.

Once part A, the at least one antimicrobial and, optionally, any additives have been sufficiently mixed to form a substantially homogeneous mixture, part B may be added to the first substantially homogeneous mixture and mixed to form a second substantially homogeneous mixture including the part A, the part B, the at least one antimicrobial, and any additives (84). Once combined, the part A and part B may begin to react and form cured silicone once combined.

Although FIGS. 6A and 6B do not show such a step, in some examples, at least one antimicrobial may be mixed into part B before mixing part A and part B to form the second substantially homogeneous mixture (84). For example, a first antimicrobial may be mixed into part A, and a second antimicrobial different from the first antimicrobial may be mixed into part B. Parts A and B, including the first and second antimicrobials and, optionally any additives, then may be mixed to form the second substantially homogeneous mixture (84). In some examples, mixing a first antimicrobial in part A and a second antimicrobial in part B may improve mixing compared to mixing two antimicrobials in part A or two antimicrobials in part B.

In some examples not illustrated in FIGS. 6A and 6B, part A and part B may be mixed first, and the antimicrobial(s) and any additives may subsequently be added to the part A and part B mixture. The antimicrobial and any additives may be mixed in an amount of part A or part B and introduced into the mixture of part A and part B, or may be mixed in another carrier, such as silicone oil, PEG, glycerol, or the like, and then mixed into the mixture of part A and part B.

As the part A and part B begin to react and form cured silicone, the mixture may be formed into a desired shape (86). The mixture may be formed into the desired shape by, for example, extrusion, injection molding, compression molding, transfer molding, casting, painting, spraying, wet film application, or the like. The desired shape may include the final form factor of antimicrobial accessory 26, or may include a shape that requires further processing to form antimicrobial accessory 26. For example, the mixture may be molded or cast into the form factor of antimicrobial accessory 26. In other examples, the mixture may be extruded, sprayed, cast, or molded into a sheet or film, which is subsequently cut or stamped into the final form factor of antimicrobial accessory 26.

In some example, the mixture may be formed into the desired shape (86) on a support material, such as a release liner. The release liner may simply provide support for the mixture while the cure is occurring, or may be part of the final packaging; that is, antimicrobial accessory 26 ultimately may be delivered to the implanting physician on the release liner.

In other examples, the mixture may have sufficient melt strength that the mixture may be formed into the desired shape (86) by extruding the mixture without support, e.g., without a release liner. The desired shape may include a sheet, film, filament, or the like.

In some examples, the mixture may be formed into the desired shape (86) in a mold or die, which provides support and defines the shape of the mixture while the mixture is curing (88).

In some examples, as shown in FIG. 6B, the mixture may optionally be processed to reduce or eliminate any bubbles in the mixture (87) after forming the mixture to the desired shape (86). In other examples, the mixture may optionally be processed to reduce or eliminate any bubbles in the mixture (87) prior to forming the mixture to the desired shape (86). For example, the mixture may be exposed to a vacuum, or may be sonicated to break up or remove the bubbles. In some cases, the reduction of removal of bubbles (87) may occur during curing of the silicone (88).

Regardless of how the mixture is formed to the desired shape, the part A and part B react to form a cured silicone (88). As described above, when the silicone is a RTV silicone, the cure may occur at approximately room temperature. In other examples, curing the silicone may occur at an elevated temperature. Alternatively, the silicone may be cured by exposure to infrared radiation. In examples in which the mixture is cured at an elevated temperature, e.g., a temperature greater than that of the surrounding room or building in which the silicone is being cured, the mixture may be exposed to a furnace. For example, the mixture may be extruded through a furnace that heats the mixture to a desired temperature for a desired length of time. In other examples, the mixture may be cured in a heated mold or die.

In some examples, the cured silicone itself may be an adhesive, e.g., a PSA, and may not require a layer of adhesive to be applied in order to adhere to ICD 16. In examples such as these, the mixture may be formed into the desired shape (86) and cured (88) on a release liner, such as a fluoropolymer release liner.

In other examples, the cured silicone does not have adhesive properties, and may have a layer of adhesive, such as a PSA, applied to a surface of the cured silicone (89) (FIG. 6B) to facilitate attachment of antimicrobial accessory 26 to housing 40 of ICD 16 (FIG. 1). In examples such as these, the mixture may be formed into the desired shape (86) on a release liner, and after the mixture cures to form cured silicone (88), the adhesive may be applied to the exposed surface of the silicone (89). In other examples, the mixture may have sufficient melt strength to allow a film of the mixture to be extruded into the desired shape (86) without support of a release liner or another supporting material. The mixture may then be cured (88) in the form of the sheet, and the adhesive may be applied to one side of the cured silicone (89). The side of the silicone having the adhesive applied thereto may then be placed on a release liner.

In still other examples, the silicone may not itself have adhesive properties and may not have an adhesive applied to a surface of the silicone. In some of these examples (not covered by the claims), the antimicrobial accessory 26 may be attached to IMD 16 by other means, such as a suture or a staple. In other of these examples (not covered by the claims), the antimicrobial accessory 26 may not be intended to be attached to the IMD 16, but may instead be implanted proximate to the IMD 16 without being attached to IMD 16.

In still other examples, the antimicrobial accessory 26 may be packaged with an amount of adhesive that is not deposited on the silicone, but is available for application to antimicrobial accessory 26 by an implanting clinician prior to attaching the accessory 26 to IDM 16. For example, antimicrobial accessory 26 may be disposed on a release liner, and an amount of adhesive may be disposed on the release liner next to antimicrobial accessory 26. This method of delivery may provide the implanting physician with greater flexibility in selecting whether to, and how to, attach antimicrobial accessory 26 to IMD 16.

Once antimicrobial accessory 26 has been formed (86) and cured (88), the accessory 26 may be packaged and sterilized (90). For example, antimicrobial accessory 26 may be packaged in a foil pouch. The foil pouch may be evacuated of air using a vacuum, or may be backfilled with an inert gas. In some examples, the foil pouch may also enclose a desiccant to trap moisture present in the foil pouch and/or may enclose an oxygen scavenging component to absorb oxygen present in the foil pouch.

Antimicrobial accessory 26 may be sterilized by, for example, electron beam sterilization, gamma beam sterilization, ethylene oxide sterilization, or autoclaving. The sterilization method may be selected to provide a efficacious sterilization of antimicrobial accessory 26 while minimizing degradation of the antimicrobial or polymer in antimicrobial accessory.

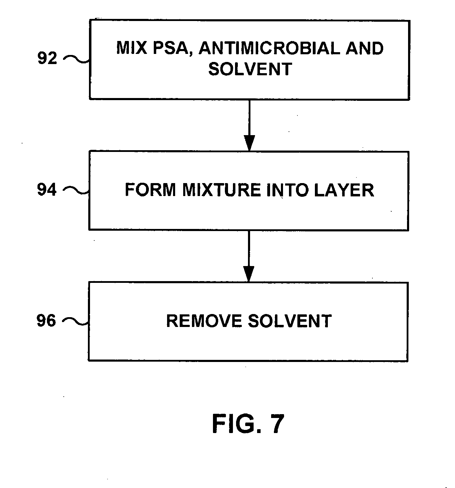

FIG. 7 is a flow diagram illustrating an example of a technique of manufacturing an antimicrobial accessory 26 including a PSA. The PSA may include, for example, a silicone PSA, an acrylic PSA, a polyurethane PSA, a polyisobutylene PSA, a cyanoacrylate PSA, a PLGA-based PSA, or the like. In some examples the PSA may be provided from the seller as solids in a solvent, e.g., 60 wt. % solids in a solvent.

First, the PSA, an antimicrobial, and a solvent may be mixed to form a first mixture (92). As described above, the antimicrobial may include at least one of a tetracycline (e.g., minocycline, doxycycline), a rifamycin (e.g., rifampin, rifaximin, rifapentine, rifabutin), a macrolide (e.g., erythromycin), a penicillin (e.g., nafcillin), a cephalosporin (e.g., cefazolin), another beta-lactam antibiotic (e.g., imipenem, aztreonam) an aminoglycoside (e.g., gentamicin), a glycopeptide (e.g., vancomycin, teicoplanin), a quinolone (e.g., ciprofloxacin), fusidic acid, trimethoprim, metronidazole, mupirocin, a polene (e.g., amphotericin B), an azole (e.g., fluconazole) and a beta-lactam inhibitor (e.g., sulbactam), tigecycline, daptomycin, clindamycin, or another fluoroquinolone, bacitracin, neomycin, an antiseptic, an antimicrobial peptide, a quaternary ammonium, or the like. In some examples, the antimicrobial may be provided in a salt form, e.g., minocycline HCl, gentamicin crobefate, or gentamicin sulfate.

The solvent may be selected based on its ability to dissolve the antimicrobial and the PSA. The solvent may include, for example, ethyl acetate, tetrahydrofuran, methanol, ethanol, acetonitrile, hexane, diethyl ether, chloroform, 1,4-dioxane, dichloromethane, acetone, dimethylformamide, dimethyl sulfoxide, acetic acid, or the like. In some examples, the antimicrobial may be dissolved in the solvent to between approximately 0.5% and approximately 20% weight per volume (i.e., between approximately 0.5 g antimicrobial per 100 mL solvent to approximately 20 g antimicrobial per 100 mL solvent). For example, the antimicrobial may be dissolved in the solvent to between approximately 1% and approximately 6% weight per volume.

While not shown in FIG. 7, in some examples, an additive also may be mixed in the mixture. As described above, the additive may include an antioxidant, which may reduce or eliminate degradation of the at least one antimicrobial due to oxidation. Exemplary antioxidants include, but are not limited to, monofunctional hindered phenolic antioxidants, such as butylated hydroxyl toluene (BHT), vitamin E, vitamin A, vitamin C, or those available under the trade designation Ciba(R) Irganox® 1076 or Ciba® Irganox® 1010, from BASF, Florham Park, N.J. In some examples, antimicrobial accessory 26 may include between approximately 0.1 wt. % and approximately 2 wt. % antioxidant.

The additive may also include an additive that affects the release rate or elution rate of the at least one antimicrobial from antimicrobial accessory 26. For example, the additive that affects the release rate may include a plasticizer or another excipient. A plasticizer or excipient may affect the viscosity of the polymer in antimicrobial accessory 26, which may in turn affect the release rate of the at least one antimicrobial. Thus, incorporation of a plasticizer or excipient may be one manner in which the time over which the antimicrobial is released from antimicrobial accessory 26 is affected. In some examples, the additive may swell or dissolve in biological fluids present in the body of patient 12, which may affect the release rate of the antimicrobial. Exemplary additives that influence the release rate of the antimicrobial accessory may include, for example, poly(acrylic acid), poly(methacrylic acid), poly(vinylpyrolidone), a sugar ester, macrogol 15 hydroxystearate (IV), poly(lactic acid), lactic acid, glycerol, PEG, methyl PEG, poly(glycolic acid), poly(ε-caprolactum), polysorbate 80 (polyoxyethylene (20) sorbitan monooleate), polysorbate 20 (polyoxyethylene (20) sorbitan monolaurate), salts such as KCl, cationic surfactants, anionic surfactants, natural surfactants, or the like. Exemplary surfactants include, but are not limited to, SDS, sodium stearate, sucrose stearate, stearyl alcohol, glycerol monostearate, mannitol, sodium laureth sulfate, sodium lauryl sulfate, triton X 100, sorbitol, fructose, chitosan, hyaluronic acid, alginate, and TMDA. As another example, antimicrobial accessory 26 may include fumed silica. Fumed silica may increase polymer integrity and may facilitate faster release of the antimicrobial. In some examples, the additive that influences the release rate of the antimicrobial accessory may constitute less than approximately 1 weight percent (wt. %) of antimicrobial accessory 26.

The mixture may be formed into a layer (94). For example, the mixture may be formed into the layer on a release liner. The layer may be formed by spray coating the substantially homogeneous mixture on the liner, air knife coating, gap coating, gravure coating, knife coating, slot die coating, metering rod coating, or the like. The formed layer of the mixture is then heated or exposed to a lower pressure to remove substantially all of the solvent from the mixture and form an antimicrobial including the PSA and the first and second antimicrobials (96).

In some examples, the PSA including the first and second antimicrobials may require further manipulation to result in the antimicrobial accessory 26. For example, the antimicrobial accessory 26 may be cut or stamped out of the PSA layer. For example, a disk (e.g., disk 28, FIG. 1) disposed on a release liner may be stamped out of the PSA layer disposed on the release liner. As another example, the PSA layer may be formed into a sleeve 62 (FIG. 4) or pouch 72 (FIG. 5).

Finally, antimicrobial accessory 26 may be packaged and sterilized (90), as described above with reference to FIGS. 6A and 6B. For example, antimicrobial accessory 26, along with a desiccant, may be packed in a foil package and sterilized using electron beam, gamma beam, or ethylene oxide sterilization.

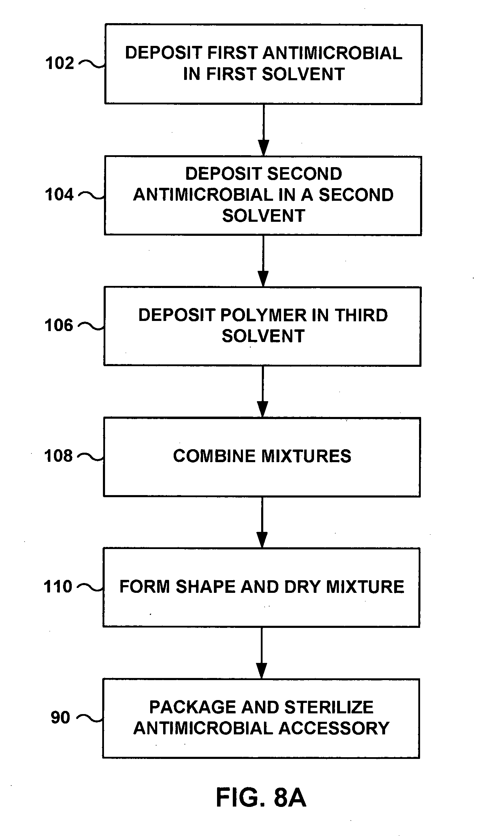

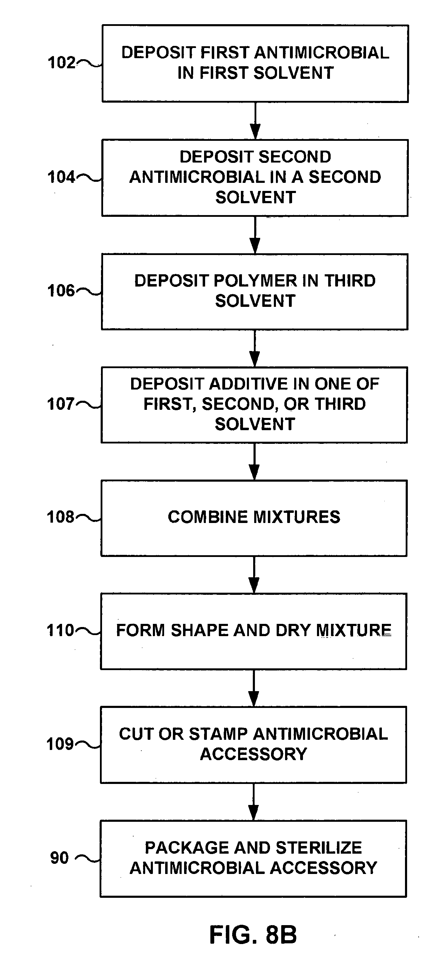

FIGS. 8A and 8B are flow diagrams of exemplary techniques of manufacturing an antimicrobial accessory 26 from a polymer that is already cured, or which does not require curing. The polymer may include, for example, a PSA, such as a silicone PSA, a polyurethane PSA, an acrylic PSA, a polyisobutylene PSA, a cyanoacrylate PSA, a PLGA-based PSA, or the like. In some examples the PSA may be provided from the seller as solids in a solvent, e.g., 60 wt. % solids in a solvent. While the examples of FIGS. 8A and 8B are described with reference to a silicone PSA, in other examples, the techniques may be utilized to form an antimicrobial accessory 26 from another PSA, such as a an acrylic PSA, a polyurethane PSA, a polyisobutylene PSA, a cyanoacrylate PSA, a PLGA-based PSA, or another polymer. In addition, the techniques of FIGS. 8A and 8B may be utilized to form an antimicrobial accessory 26 including a biodegradable or bioabsorbable polymer, such as, for example, collagen, PLGA, PLA, PGA, PEO, POE, PCL, poly(dioxanone), polyglyconate, hyaluronic acid, gelatin, fibrin, fibrinogen, cellulose, starch, cellulose acetate, PVP, a PEO/PPO copolymer, poly(ethylene vinyl acetate), poly(hydroxybutyrate-covalerate), polyanhydride, poly(glycolic acid-co-trimethylene carbonate), polyphosphoester, polyphosphoester urethane, a poly(amino acid), a cyanoacrylate, poly(trimethylene carbonate), poly(iminocarbonate), a copoly(ether-ester) such as PEO/PLA, a polyalkylene oxalate, a polyphasphazene, a polyarylate, a tyrosine-based biodegradable or bioabsorbable polymer, PHA, a sugar ester, or the like.

First, a first antimicrobial may be deposited or dissolved in a first solvent (102) to form a first mixture. The first antimicrobial may include, for example, an antibiotic such as a tetracycline (e.g., minocycline, doxycycline), a rifamycin (e.g., rifampin, rifaximin, rifapentine, rifabutin), a macrolide (e.g., erythromycin), a penicillin (e.g., nafcillin), a cephalosporin (e.g., cefazolin), another beta-lactam antibiotic (e.g., imipenem, aztreonam) an aminoglycoside (e.g., gentamicin), a glycopeptide (e.g., vancomycin, teicoplanin), a quinolone (e.g., ciprofloxacin), fusidic acid, trimethoprim, metronidazole, mupirocin, a polene (e.g., amphotericin B), an azole (e.g., fluconazole) and a beta-lactam inhibitor (e.g., sulbactam), tigecycline, daptomycin, clindamycin, or another fluoroquinolone, bacitracin, neomycin, an antiseptic, an antimicrobial peptide, a quaternary ammonium, or the like. In some examples, the antimicrobial may be provided in a salt form, e.g., minocycline HCl, gentamicin crobefate, or gentamicin sulfate. The first solvent may be selected according to its ability to dissolve the first antimicrobial, and may include, for example, ethyl acetate, tetrahydrofuran, methanol, ethanol, acetonitrile, hexane, diethyl ether, chloroform, 1,4-dioxane, dichloromethane, acetone, dimethylformamide, dimethyl sulfoxide, acetic acid, or the like. For example, rifampin may be dissolved in ethyl acetate or tetrahydrofuran, and minocycline may be dissolved methanol or ethanol. In some examples, the antimicrobial may be dissolved in the solvent to between approximately 0.5% and approximately 20% weight per volume (i.e., between approximately 0.5 g antimicrobial per 100 mL solvent to approximately 20 g antimicrobial per 100 mL solvent). For example, the antimicrobial may be dissolved in the solvent to between approximately 1% and approximately 6% weight per volume.

A second antimicrobial may be deposited or dissolved in a second solvent (104) to form a second mixture. The second antimicrobial may include, for example, an antibiotic such as a tetracycline (e.g., minocycline, doxycycline), a rifamycin (e.g., rifampin, rifaximin, rifapentine, rifabutin), a macrolide (e.g., erythromycin), a penicillin (e.g., nafcillin), a cephalosporin (e.g., cefazolin), another beta-lactam antibiotic (e.g., imipenem, aztreonam) an aminoglycoside (e.g., gentamicin), a glycopeptide (e.g., vancomycin, teicoplanin), a quinolone (e.g., ciprofloxacin), fusidic acid, trimethoprim, metronidazole, mupirocin, a polene (e.g., amphotericin B), an azole (e.g., fluconazole) and a beta-lactam inhibitor (e.g., sulbactam), tigecycline, daptomycin, clindamycin, or another fluoroquinolone, bacitracin, neomycin, an antiseptic, an antimicrobial peptide, a quaternary ammonium, or the like. In some examples, the antimicrobial may be provided in a salt form, e.g., minocycline HCl, gentamicin crobefate, or gentamicin sulfate. The second antimicrobial may be different than the first antimicrobial. The solvent may include, for example, ethyl acetate, tetrahydrofuran, methanol, ethanol, acetonitrile, hexane, diethyl ether, chloroform, 1,4-dioxane, dichloromethane, acetone, dimethylformamide, dimethyl sulfoxide, acetic acid, or the like, and may be different than the first solvent. For example, the first solvent may include ethanol or methanol and the first antimicrobial may include minocycline, while the second solvent includes ethyl acetate and the second antimicrobial includes rifampin.

In addition, a polymer may be dissolved in a third solvent (106) to form a third mixture. In some examples, the third solvent is different from the first solvent and different from the second solvent. In other examples, third solvent may be the same as the second solvent. The third solvent may include, for example, ethyl acetate, tetrahydrofuran, methanol, ethanol, acetonitrile, hexane, diethyl ether, chloroform, 1,4-dioxane, dichloromethane, acetone, dimethylformamide, dimethyl sulfoxide, acetic acid, or the like. In one example, the polymer includes a silicone pressure sensitive adhesive, the first antimicrobial includes minocycline, the second antimicrobial includes rifampin, the first solvent includes ethanol or methanol, and the second and third solvents include ethyl acetate. In some examples, the polymer and the second antimicrobial may be dissolved in the same batch of solvent, e.g., the polymer may be provided in a solvent and the second antimicrobial may be dissolved directly into the polymer/solvent mixture. In some examples, the polymer may be dissolved in the solvent to between approximately 1% and approximately 80% weight per volume (i.e., between approximately 1 g polymer per 100 mL solvent to approximately 80 g polymer per 100 mL solvent), or approximately 60% weight per volume.

In some examples, as shown in FIG. 7B, an additive may be dissolved in one or more of the first, second, or third solvents (107). As described above, the additive may include an antioxidant, which may reduce or eliminate degradation of the at least one antimicrobial due to oxidation. Exemplary antioxidants include, but are not limited to, monofunctional hindered phenolic antioxidants, such as butylated hydroxyl toluene (BHT), vitamin E, vitamin A, vitamin C, or those available under the trade designation Ciba® Irganox® 1076 or Ciba® Irganox® 1010, from BASF, Florham Park, N.J. In some examples, antimicrobial accessory 26 may include between approximately 0.1 wt. % and approximately 2 wt. % antioxidant. The additive may also include an additive that affects the release rate or elution rate of the at least one antimicrobial from antimicrobial accessory 26. For example, the additive that affects the release rate may include a plasticizer or another excipient. A plasticizer or excipient may affect the viscosity of the polymer in antimicrobial accessory 26, which may in turn affect the release rate of the at least one antimicrobial. Thus, incorporation of a plasticizer or excipient may be one manner in which the time over which the antimicrobial is released from antimicrobial accessory 26 is affected. In some examples, the additive may swell or dissolve in biological fluids present in the body of patient 12, which may affect the release rate of the antimicrobial. Exemplary additives that influence the release rate of the antimicrobial accessory may include, for example, poly(acrylic acid), poly(methacrylic acid), poly(vinylpyrolidone), a sugar ester, macrogol 15 hydroxystearate (IV), poly(lactic acid), lactic acid, glycerol, PEG, methyl PEG, poly(glycolic acid), poly(ε-caprolactum), polysorbate 80 (polyoxyethylene (20) sorbitan monooleate), polysorbate 20 (polyoxyethylene (20) sorbitan monolaurate), salts such as KCl, cationic surfactants, anionic surfactants, natural surfactants, or the like. Exemplary surfactants include, but are not limited to, SDS, sodium stearate, sucrose stearate, stearyl alcohol, glycerol monostearate, mannitol, sodium laureth sulfate, sodium lauryl sulfate, triton X 100, sorbitol, fructose, chitosan, hyaluronic acid, alginate, and TMDA. As another example, antimicrobial accessory 26 may include fumed silica. Fumed silica may increase polymer integrity and may facilitate faster release of the antimicrobial. In some examples, the additive that influences the release rate of the antimicrobial accessory may constitute less than approximately 1 weight percent (wt. %) of antimicrobial accessory 26.

The first, second, and third mixtures may then be combined and mixed together to form a substantially homogeneous mixture including the first and second antimicrobials and the polymer (108). The mixtures may be mixed in a wide variety of mixing apparatuses, including, for example, a static mixer, dental speed mixer, Brabender mixer, or the like. In some examples, the first, second, and third mixtures may be heated to facilitate mixing and lessen the risk of the polymer or antibiotic precipitating from the mixture.

Once the first, second, and third mixtures have been mixed into a substantially homogeneous mixture, the substantially homogeneous mixture may be formed into a desired shape and dried to remove substantially all of the solvent (110). For example, the substantially homogeneous mixture maybe formed into a layer on a release liner. The layer may be formed by spray coating the substantially homogeneous mixture on the liner, air knife coating, gap coating, gravure coating, knife coating, slot die coating, metering rod coating, or the like. The formed layer of the mixture is then heated or exposed to a lower pressure to remove substantially all of the solvents from the mixture and form a PSA including the first and second antimicrobials.

In some examples, the PSA including the first and second antimicrobials may require further manipulation to result in the antimicrobial accessory 26. For example, as shown in FIG. 8B, the antimicrobial accessory 26 may be cut or stamped out of the PSA layer (109). For example, a disk (e.g., disk 28, FIG. 1) disposed on a release liner may be stamped out of the PSA layer disposed on the release liner. As another example, the PSA layer may be formed into a sleeve 62 (FIG. 4) or pouch 72 (FIG. 5).

Finally, antimicrobial accessory 26 may be packaged and sterilized (90), as described above with reference to FIGS. 6A and 6B. For example, antimicrobial accessory 26, along with a desiccant, may be packed in a foil package and sterilized using electron beam, gamma beam, or ethylene oxide sterilization.

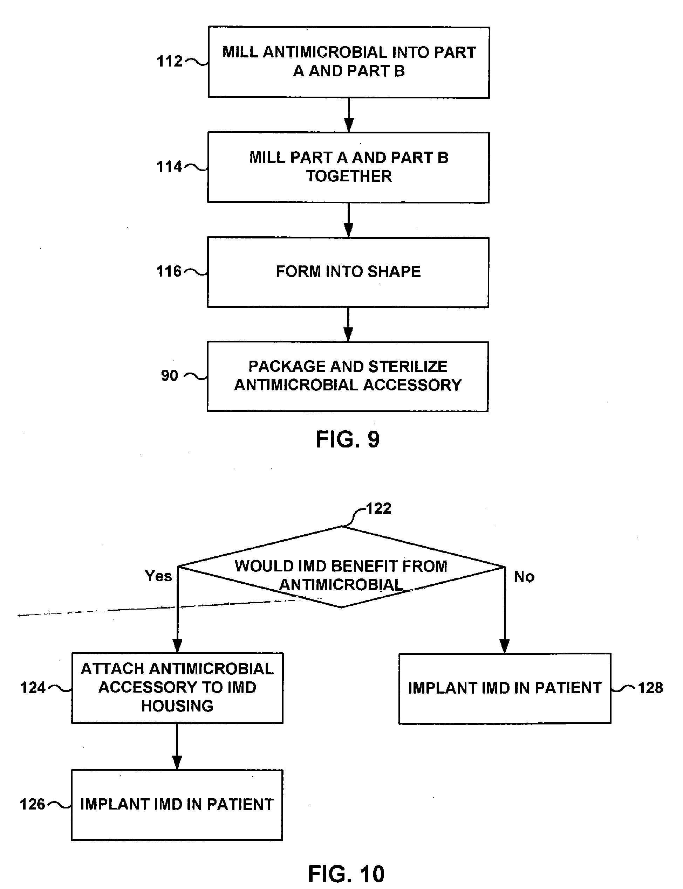

FIG. 9 is a flow diagram of an example method of forming an antimicrobial accessory including an enhanced tear resistance (ETR) silicone. Exemplary ETR silicones include, for example, those with part numbers Q7-4735, Q7-4750, or Q7-4765 from Dow Coming, Midland, MI. An ETR silicone may be provided as first and second constituent parts ("part A" and "part B"); however, in some examples the first and second constituent parts may not be pumpable. An ETR silicone such as this may be processed by milling or another high shear mixing apparatus, such as a Brabender mixer. First, the at least one antimicrobial may be mixed separately into each of the parts A and B (112), using for example, a Brabender mixer or a chilled two roll mill. Parts A and B, which each include the at least one antimicrobial, may then be mixed together using a similar or different high shear mixing technique (114). The ETR silicone mixed with the antimicrobial may then be processed at an elevated temperature to form the ETR silicone into a desired shape (116). For example, the ETR silicone including the antimicrobial may be extruded or molded at an elevated temperature, to cause parts A and B to react and begin curing the ETR silicone. The ETR silicone may be formed into a desired shape, which may be a sheet, disk, film, or the like, and fully cured. In some examples, the ETR silicone may be cut or stamped to the final form factor of antimicrobial accessory 26, as described above, and may be provided with an adhesive on a surface of the antimicrobial accessory 26, also described above. Finally, antimicrobial accessory 26 including the ETR silicone may be packaged and sterilized (90).

In some examples, a biodegradable or bioabsorbable polymer may be heated to form a polymer melt, and the antimicrobial may be mixed in the polymer melt. The polymer and antimicrobial may then be processed by thermoplastic processing methods, such as static mixing, twin-screw or single-screw extrusion, molding, casting, and the like, to form antimicrobial accessory. The time for which the polymer and antimicrobial are processed at an elevated temperature may be selected or controlled to limit degradation of the antimicrobial.

In some examples, an RTV silicone may be formed from one component that cures at room temperature to form the cured silicone. In examples such as these, at least one antimicrobial may be mixed into the uncured RTV silicone to form a substantially homogeneous mixture, either with or without a solvent. The mixture may then be cured by exposure to atmospheric moisture, e.g., water vapor. Some such RTV silicone systems may include acetoxy, methoxy or ethoxy groups that react with moisture vapor and liberate acetic acid, methanol or ethanol, respectively, during the curing process. As the RTV silicone cures, the mixture may be formed to the desired form factor of the antimicrobial accessory.

FIG. 10 is a flow diagram illustrating a technique for determining whether an IMD 16 may benefit from being implanted with an antimicrobial accessory 26. First, the implanting clinician may consider whether patient 12 is a would benefit from IMD 16 being implanted with an antimicrobial accessory 26 adhered to IMD 16.

In determining whether patient 12 would benefit from antimicrobial accessory 26, the implanting clinician may consider risk factors, such as, for example, previous or expected device change-outs, previous surgical procedures, comorbidities such as renal failure, renal dialysis, or diabetes, an infection rate at the implanting center or hospital, a state of the immune system of patient 12, or the like. Based on the risk of infection, the implanting clinician may determine to utilize or not to utilize antimicrobial accessory 26. In some examples, the implanting clinician may not consider risk factors and may utilize antimicrobial accessory 26 in all cases.

When the implanting physician determines that the patient 12 is a candidate for implantable of IMD 16 with antimicrobial accessory 26, the implanting physician may adhere antimicrobial accessory 26 to housing 40 of IMD 16 (124). For example, as described above, antimicrobial accessory 26 may be formed of a PSA or may include an adhesive applied to a surface of a polymer layer. In these examples, antimicrobial accessory 26 may be adhered to housing 40 of IMD 16 by the implanting physician. In other examples (not claimed), the implanting physician may suture or staple antimicrobial accessory 26 to IMD 16 (e.g., connector block 27, FIG. 1). The implanting physician may then implant the IMD 16 and antimicrobial accessory in the body of patient 12 (126).

When the implanting physician determines that patient 12 is not likely to benefit from antimicrobial accessory 26, e.g., if patient 12 has a low risk of contracting a post-implant infection, the implanting physician may implant IMD 16 in patient 12 without antimicrobial accessory 26 (128).

The antimicrobial accessory 26 may be provided to the implanting physician in different ways. For example, antimicrobial accessory 26 may be provided alone, and may be configured to be used with a variety ofIMDs, such as different models of ICDs, pacemakers, drug delivery devices, neurostimulators, or monitoring devices. The implanting physician may determine that a patient 12 may benefit from antimicrobial accessory and may attach antimicrobial accessory 26 to IMD 16 prior to implanting IMD 16 in patient 12.

In other examples, antimicrobial accessory 26 may be bundled together in a kit with an IMD 16, but may be provided physically separately, e.g., may require the implanting clinician to attach antimicrobial accessory 26 to IMD 16 before implantation. This may provide convenience of having an antimicrobial accessory 26 provided with an IMD 16, but may still permit an implanting clinician to elect if he or she wishes to utilize the antimicrobial accessory 26 on a patient-by-patient basis.

In other examples, an IMD 16 may be provided to the implanting clinician with antimicrobial accessory 26 already attached. This may provide the most straightforward implementation, as the implanting physician is not required to decide whether the antimicrobial accessory 26 is desired, and does not need to attach antimicrobial accessory 26 to IMD 16 prior to implanting IMD 16 in patient 12.

In some examples, the materials (polymers, antimicrobials, additives such as antioxidants, plasticizers or other excipients, surfactants, or the like) described herein may be used in a patterned antimicrobial accessory. A patterned antimicrobial accessory may provide benefits. For example, a patterned antimicrobial may facilitate use of two or more antimicrobials in a single antimicrobial accessory. In some cases, systems that include two or more antimicrobials mixed in a single polymer portion (i.e., not patterned) may have compromised chemical or physical properties. For example, the at least two antimicrobials may not be chemically compatible and/or may degrade more quickly when mixed. Alternatively, one or more of the at least two antimicrobials may require an excipient (or additive), such as an antioxidant, for stability, and the additive or excipient may not be compatible with another one of the antimicrobials. As another example, the mechanical stability of the polymer may be reduced by the inclusion of at least two antimicrobials, because the combined concentration of the antimicrobials may be sufficiently high to affect the mechanical properties of the polymer. Additionally or alternatively, the two antimicrobials may have different release rates from the antimicrobial accessory due to, for example, different interactions with the polymer (e.g., different solubilities in the polymer and/or the surrounding environment).

By separating the two or more antimicrobials into different domains in a patterned antimicrobial accessory, at least one of the problems described above may be mitigated or eliminated. For example, separating the at least two antimicrobials into separate domains may eliminate compatibility issues between the antimicrobials, and may allow independent selection of the excipients included with each of the at least two antimicrobials.

Additionally or alternatively, a patterned antimicrobial accessory may facilitate selection of polymer/antimicrobial/excipient combinations that provide desired release rates for each of the antimicrobials. The desired release rate may be the same or different for each antimicrobial. For example, polymer/antimicrobial/excipient combinations may be selected to provide substantially similar release profiles for each of the at least two antimicrobials in examples in which the antimicrobials beneficially interact to be more efficacious than one or more of the antimicrobials used alone. As another example, polymer/antimicrobial/excipient combinations may be selected to provide relatively short-term release of a first antimicrobial and relatively long-term release of a second antimicrobial.

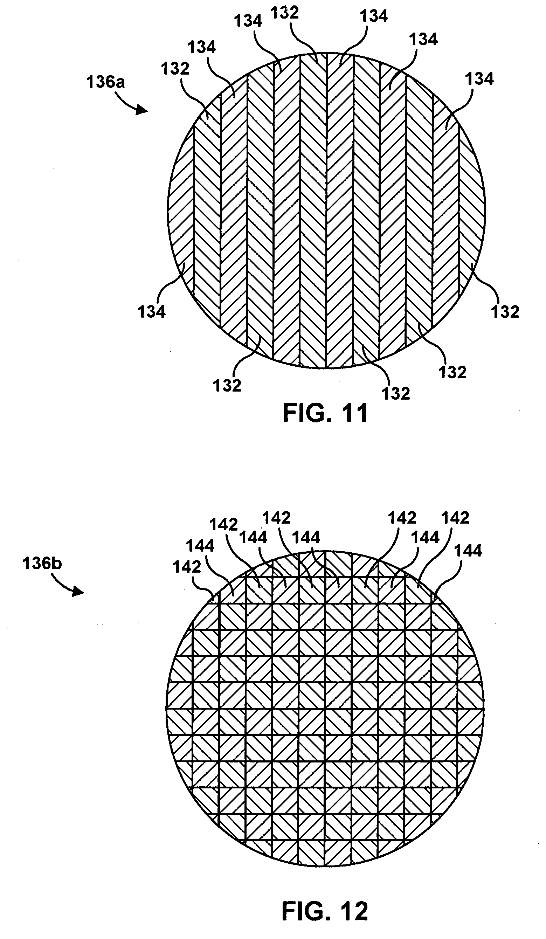

FIG. 11 illustrates an example of a patterned antimicrobial accessory 136a including a pattern of first domains 132 and second domains 134. In the example shown in FIG. 11, the first domains 132 and second domains 134 are arrayed as a plurality of substantially parallel strips. First domains 132 and second domains 134 are substantially contiguous with each other, i.e., there is no intervening material or space between an edge of one of first domains 132 and an edge of an adjacent one of second domains 134. First domains 132 and second domains 134 may include a different composition of polymer, antimicrobial and/or excipient. In some examples, first domains 132 and second domains 134 may include the same polymer, different antimicrobials, and the same or different excipients. For example, each of first domains 132 and second domains 134 may include silicone. First domains 132 may include minocycline HCl, while second domains 134 may include rifampin. Additionally, at least one of first domains 132 and second domains 134 may include an excipient, such as an antioxidant or a plasticizer. In some examples, first domains 132 and second domains 134 include the same excipient, while in other examples, first domains 132 include a different excipient than second domains 134. Alternatively, one of first domains 132 or second domains 134 may include an excipient and the other may not.

In other examples, first domains 132 and second domains 134 may include different polymers and the same antimicrobial, which may provide different release rates from the first domains 132 and the second domains 134. The combination of release rates from first domains 132 and second domains 134 may enable desirable release profiles. For example, first domains 132 may include a polymer which results in relatively fast burst elution of the antimicrobial, while second domains 134 includes a polymer which results in longer-term sustained release of the antimicrobial. In this way, a relatively high initial concentration of the antimicrobial may be provided soon after implantation of patterned antimicrobial accessory 136a with a lower concentration of antimicrobial provided for a longer time after implantation.

For example, first domains 132 may include a PSA, such as a silicone PSA, a polyurethane PSA, an acrylic PSA, a polyisobutylene PSA, a cyanoacrylate PSA, a PLGA-based PSA, or the like. Second domains 134, then, may include another polymer, which may or may not be a PSA. In some examples, first domains 132 may comprise a PSA and second domains 134 may comprise a polymer that is not an adhesive. In this way, first domains 132 may be used to adhere antimicrobial accessory 136a to a housing of an IMD, such as ICD 16 (FIG. 1). Additionally, first domains 132 may carry an antimicrobial. As described above, an elution rate of an antimicrobial from a PSA may be greater than an elution rate of an antimicrobial form a polymer having a higher crosslink density, because the PSA may have little or no crosslinking. Thus, first domains 132 may be used to adhere antimicrobial accessory 136a to an IMD and may also facilitate control of an elution rate of antimicrobial from antimicrobial accessory 136a.

In some examples of an antimicrobial accessory 136a in which first domains 132 comprise a PSA, second domains 134 comprising a polymer which is not a PSA may also provide beneficial properties to accessory 136a. For example, some polymers used in second domains 134 may have greater mechanical integrity than a PSA used in first domains 132, and may contribute the mechanical properties (e.g., strength) of antimicrobial accessory 136a. In this way, an antimicrobial accessory including a polymer that is not a PSA in second domains 134 may have improved mechanical properties, and may be easier to handle, than an antimicrobial accessory 136a including PSAs in both first domains 132 and second domains 134.

In some examples, first domains 132 and second domains 134 may include different polymers and different antimicrobials. For example, first domains 132 may include a first polymer and a first antimicrobial and second domains 134 may include a second polymer different than the first polymer and a second antimicrobial different than the first antimicrobial. As described above, the first and second polymers may be selected to provide substantially similar release rates for the first and second antimicrobials, or may be selected to provide different release rates for the first and second antimicrobials. In some examples, at least one of first domains 132 and second domains 134 may include a PSA, such as a silicone PSA, a polyurethane PSA, an acrylic PSA, a polyisobutylene PSA, a cyanoacrylate PSA, a PLGA-based PSA, or the like.

Although FIG. 11 has been described with reference to first domains 132 and second domains 134, in some examples, patterned antimicrobial accessory 136a may include more than two domains. For example, patterned antimicrobial accessory 136a may include at least three domains, which may each include similar or different polymers, similar or different antimicrobials, and/or similar or different excipients, such as plasticizers or antioxidants.

Additionally or alternatively, while FIG. 11 includes first domains 132 and second domains in a pattern of alternating, substantially parallel strips, in other examples, patterned antimicrobial accessory 136a may include third domains (not shown) that are interspersed between a first domains 132 and a respective one of second domains 134. The third domains may separate respective first domains 132 and the respective second domains 134 by introducing strips of polymer that do not include antimicrobial between first domains 132 and second domains 134.

FIG. 12 illustrates another example of a patterned antimicrobial accessory 136b, which includes first domains 142 and second domains 144 arranged in a checkerboard array. Similar to FIG. 11, first domains 142 and second domains 144 are substantially contiguous, i.e., are located immediate adjacent each other with no intervening space or material. As described above with reference to FIG. 11, first domains 142 and second domains 144 may include the same or different polymers, the same or different antimicrobials, and/or the same or different excipients. Any of the materials described above may be used in either or both of first domains 142 and second domains 144.

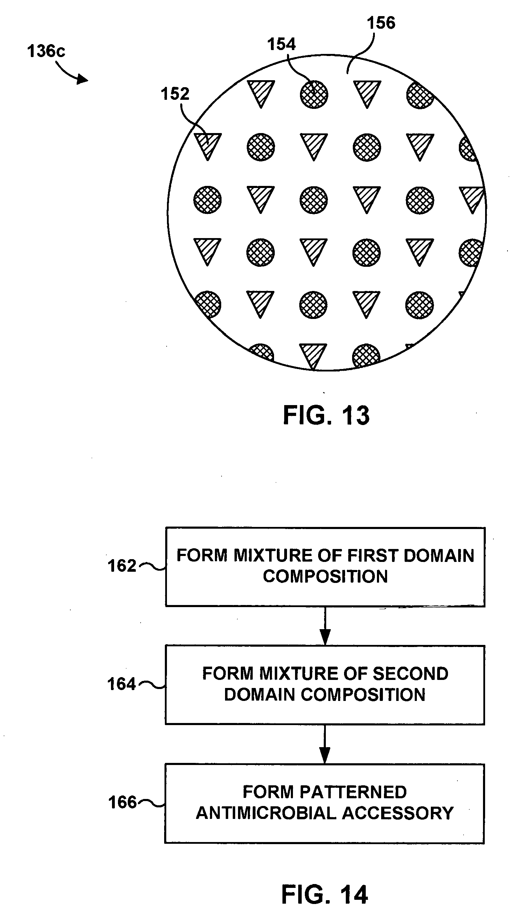

In other examples, as shown in FIG. 13, a patterned antimicrobial accessory 136c may include first domains 152 and second domains 154 separated by a third domain 156. In FIG. 13, third domain 156 is substantially continuous, and forms a matrix in which distinct islands of first domains 152 and second domains 154 are formed. In some examples, first domains 152 may comprise a different shape than second domains 154. For example, first domains 152 may comprise a plurality of triangular islands and second domains 154 comprise a plurality of circular islands. Other shapes are also possible, such as, for example, tetrahedron, other polygons, ellipses, lines, or the like.

Selecting different shapes for first domains 152 and second domains 154 may contribute to control over the release rate of the antimicrobial from the respective first domains 152 or second domains 154. For example, one characteristic that may affect the release rate or release profile is the ratio of the surface area of the domain to the volume of the domain. Increasing this ratio (e.g., greater surface area for the same volume) may increase the release rate, while decreasing this ratio (e.g., greater volume for the same surface area) may decrease the release rate. In this way, the shape of first domains 152 and second domains 154 may afford further control of the release rate antimicrobial(s) in first domains 152 and second domains 154.

Third domain 156 may include a polymer and substantially no antimicrobial. Third domain 156 separates first domains 152 from second domains 154 and may substantially prevent interdiffusion of the antimicrobial in first domains 152 and the antimicrobial in second domains 154.

Although FIG. 13 illustrates regular spacing of first domains 152 and second domains 154, in other examples, the spacing between first domains 152 and second domains 154 may be different at different locations on patterned antimicrobial accessory 136c. For example, the spacing between adjacent domains 152 and/or 154 may affect the concentration of the antimicrobial when released from the domains 152 and/or 154 (e.g., a closer spacing of the domains may result in a higher concentration of antimicrobial, while a greater spacing between adjacent domains may result in a lower concentration of antimicrobial). In this way, spacing of adjacent domains is another variable that may affect the efficacy of patterned antimicrobial accessory 136c.

In any of the examples described above, the patterned antimicrobial accessory may be formed by a variety of processes. FIG. 14 is a flow diagram illustrating an example of a technique for forming a patterned antimicrobial accessory. In FIG. 14, a mixture of the first domain composition is first formed (162). The first domain composition may be formed according to any number of techniques, including, for example, techniques described above with respect to FIGS. 6A-9. In some examples, the polymer, antimicrobial, and any additives in the first domain composition may be deposited in at least one solvent. In other examples, the antimicrobial and any additives in the first domain composition may be mixed into one or more parts of a multipart uncured polymer, e.g., a silicone formed by mixing a part A and a part B. In other examples, the polymer, antimicrobial, and any additives in the first domain composition may be milled together to form the mixture.

A mixture of the second domain composition is also formed (164). In some examples, the second domain composition may be formed using the same technique as the first domain composition. In other examples, the second domain composition may be formed using a different technique that the first domain composition. Again, the second domain composition may be formed by a variety of techniques, including, for example, those described above with respect to FIGS. 6A-9.

The technique continues with forming the patterned antimicrobial accessory 136 (166). In some examples, patterned antimicrobial accessory 136 may be formed by coating a patterned substrate. The substrate may be molded into a negative of the desired pattern. The first domain composition (e.g., the composition of first domains 132) may be coated onto the patterned substrate, filling in the depressions in the patterned substrate. The second domain composition (e.g., the composition of second domains 134) is then coated onto the first coating, filling in depressions and creating the desired pattern. In other examples, the pattern may be created by screen printing, spray coating, or the like, using a screen or mask that imparts the desired pattern.

In other examples, patterned antimicrobial accessory 136 may be formed using a coating die that has at least two ports and different compositions pumped into the ports. For example, the first domain composition (e.g., the composition of first domains 132) may be pumped through a first port and the second domain composition (e.g., the composition of second domains 134) may be pumped through a second port. The compositions may be deposited onto a release liner to form the desired pattern. The patterned object may then be worked (e.g., cut) into the desired geometry of a patterned antimicrobial accessory (e.g., patterned antimicrobial accessory 136a).

In some examples, forming patterned antimicrobial accessory 136 (166) includes curing the polymer in the first domain composition or the second domain composition. For example, when the first domain composition and/or the second domain composition includes a multipart silicone, the uncured parts A and B may be exposed to an elevated temperature or a catalyst to cure and form the silicone. As another example, RTV silicone may be left at room temperature for a time to cure.

In some examples, forming patterned antimicrobial accessory 136 (166) includes removing a solvent from at least one of the first domain composition or the second domain composition. For example, when the first domain composition includes a PSA, the PSA, antimicrobial and any additive may be mixed in a solvent, and the solvent may be removed to form the patterned antimicrobial accessory 136 (166). In some embodiments, the first domain composition includes a composition that requires removal of a solvent to form patterned antimicrobial accessory 136 (166), while the second domain composition includes a composition that requires curing to form patterned antimicrobial accessory 136 (166), or vice versa.

Although not shown in FIG. 14, in some examples, a technique for forming an patterned antimicrobial accessory 136 may include one or more additional, optional steps, such as, for example, cutting or stamping the antimicrobial accessory 136 to a desired shape (see (109) FIG. 8B) or packaging and sterilizing the patterned antimicrobial accessory 136 (see (90) FIG. 6A).

### EXAMPLES

### Reference Example 1:

In this example, silicone disks including a two-part liquid silicone rubber (available under the trade designation Silastic ® MDX4-4210 from Dow Coming Corp., Midland, MI) were formed. First, 9.068 g of Part A was measured into a disposable plastic mixing cup. Part B (0.907 g) was added to part A, and the two parts were mixed with a stainless steel spatula. The combined mixture was placed under a vacuum to remove air bubbles in the mixture. Aliquots of approximately 0.43 g were deposited into 44 mm diameter aluminum pans. The silicone was cured at room temperature for approximately 48 hours. The silicone self-leveled but did not flow completely. The samples were approximately 1 mm thick instead of approximately 0.25 mm, which was expected had the silicone flowed to levelly fill the aluminum pans.

### Reference Example 2:

In this example, silicone disks including Silastic® MDX4-4210 were formed using a different procedure than example 1. First, 9.235 g of Part A was measured into a disposable plastic mixing cup. Part B (0.935 g) was added to part A, and the two parts were mixed with a stainless steel spatula. Aliquots of approximately 0.43 g were deposited into 44 mm diameter aluminum pans. The aliquots were spread over the entire bottom of the pans as evenly as possible using the flat end of the spatula. The samples were placed under a vacuum to remove air bubbles from the samples, and then cured overnight at room temperature.

### Reference Example 3:

In this example, silicone disks including Silastic® MDX4-4210, minocycline HCl, and rifampin were formed. First, 17.961 g Part A was measured into a disposable plastic mixing cup. Minocycline HCl (101.26 mg) and rifampin (103 mg) were then mixed into the Part A. Part B (1.803 g) was added to the mixture of Part A, minocycline HCl, and rifampin, and the mixture was stirred with a stainless steel spatula. Aliquots of approximately 0.87 g were deposited into 44 mm diameter aluminum pans. The aliquots were spread over the entire bottom of the pans as evenly as possible using the flat end of the spatula. The samples were cured over a weekend at room temperature. Adding minocycline HCl and rifampin made the silicone more viscous and more difficult to spread in the pans. Table 1 shows the approximate amount of minocycline HCl and rifampin in each of the samples produced in Example 3.

**Table 1: Samples 1-20 of Example 3**

| **Sample** | **Mass (g)** | **Thickness (mm)** | **Minocycline HCl mass (theoretical) (mg)** | **Rifampin mass (theoretical) (mg)** |
|---|---|---|---|---|
| 1 | 0.147 | 0.38, 0.38 | 0.744 | 0.759 |
| 2 | 0.202 | 0.45, 0.42 | 1.022 | 1.042 |
| 3 | 0.155 | 0.39, 0.35 | 0.784 | 0.800 |
| 4 | 0.182 | 0.42; 0.62 | 0.921 | 0.939 |
| 5 | 0.215 | 0.42,0.21 | 1.088 | 1.109 |
| | | | | |
| 6 | 0.188 | 0.35, 0.42 | 0.951 | 0.970 |
| 7 | 0.188 | 0.42, 0.42 | 0.951 | 0.970 |
| 8 | 0.212 | 0.49,0.41 | 1.073 | 1.094 |
| 9 | 0.189 | 0.38, 0.55 | 0.956 | 0.975 |
| 10 | 0.178 | 0.21,0.55 | 0.901 | 0.918 |
| | | | | |
| 11 | 0.163 | 0.28, 0.44 | 0.825 | 0.841 |
| 12 | 0.184 | 0.33, 0.63 | 0.931 | 0.949 |
| 13 | 0.168 | 0.40, 0.47 | 0.850 | 0.867 |
| 14 | 0.188 | 0.62, 0.32 | 0.951 | 0.970 |
| 15 | 0.184 | 0.59, 0.49 | 0.931 | 0.949 |
| | | | | |
| 16 | 0.150 | 0.21, 0.60 | 0.759 | 0.774 |
| 17 | 0.165 | 0.37, 0.61 | 0.835 | 0.851 |
| 18 | 0.182 | 0.38, 0.57 | 0.921 | 0.939 |
| 19 | 0.179 | 0.67, 0.41 | 0.906 | 0.924 |
| 20 | 0.202 | 0.54, 0.35 | 1.022 | 1.042 |

### Reference Example 4:

In this example, silicone disks including Silastic® MDX4-4210, minocycline HCl, and rifampin were formed. First, 13.638 g Part A was measured into a disposable plastic mixing cup. Minocycline HCl (approximately 2500 mg) and rifampin (approximately 2500 mg) were then mixed into the Part A. Part B (1.35 g) was added to the mixture of Part A, minocycline HCl, and rifampin, and the mixture was stirred with a stainless steel spatula. Aliquots of approximately 0.87 g were deposited into 44 mm diameter aluminum pans. The aliquots were spread over the entire bottom of the pans as evenly as possible using the flat end of the spatula. The samples were cured over a weekend at room temperature. Table 2 shows the approximate amount of minocycline HCl and rifampin in each of the samples produced in Example 4.

**Table 2: Samples 1-19 of Example 4**

| **Sample** | **Mass (g)** | **Minocycline HCl mass (theoretical) (mg)** | **Rifampin mass (theoretical) (mg)** |
|---|---|---|---|
| 1 | 0.101 | 12.633 | 12.633 |
| 2 | 0.119 | 14.884 | 14.884 |
| 3 | 0.121 | 15.134 | 15.134 |
| 4 | 0.128 | 16.010 | 16.010 |
| 5 | 0.139 | 17.385 | 17.385 |
| | | | |
| 6 | 0.141 | 17.636 | 17.636 |
| 7 | 0.122 | 15.259 | 15.259 |
| 8 | 0.141 | 17.636 | 17.636 |
| 9 | 0.136 | 17.010 | 17.010 |
| 10 | 0.111 | 13.883 | 13.883 |
| | | | |
| 11 | 0.109 | 13.633 | 13.633 |
| 12 | 0.110 | 13.758 | 13.758 |
| 13 | 0.106 | 13.258 | 13.258 |
| 14 | 0.133 | 16.635 | 16.635 |
| 15 | 0.115 | 14.384 | 14.384 |
| | | | |
| 16 | 0.106 | 13.258 | 13.258 |
| 17 | 0.183 | 22.889 | 22.889 |
| 18 | 0.100 | 12.508 | 12.508 |
| 19 | 0.130 | 16.260 | 16.260 |

### Reference Example 5:

In this example, silicone disks including Silastic® MDX4-4210 were formed using a different procedure than examples 1 and 2. First, 19.995 g of Part A was measured into a disposable plastic mixing cup. Part B (2.012 g) was added to part A, and the two parts were mixed with a stainless steel spatula. Aliquots of approximately 0.87 g were deposited into 44 mm diameter aluminum pans. The aliquots were spread over the entire bottom of the pans as evenly as possible using the flat end of the spatula. The samples were then cured over a weekend at room temperature. Table 3 shows the masses of the resulting disks.

**Table 3: Samples 1-20 of Example 5**

| **Sample** | **Mass (g)** | **Minocycline HCl mass (theoretical) (mg)** | **Rifampin mass (theoretical) (mg)** |
|---|---|---|---|
| 1 | 0.181 | --- | --- |
| 2 | 0.191 | --- | --- |
| 3 | 0.125 | --- | --- |
| 4 | 0.161 | --- | --- |
| 5 | 0.205 | --- | --- |
| | | | |
| 6 | 0.162 | --- | --- |
| 7 | 0.140 | --- | --- |
| 8 | 0.173 | --- | --- |
| 9 | 0.183 | --- | --- |
| 10 | 0.189 | --- | --- |
| | | | |
| 11 | 0.156 | --- | --- |
| 12 | 0.152 | --- | --- |
| 13 | 0.188 | --- | --- |
| 14 | 0.147 | --- | --- |
| 15 | 0.159 | --- | --- |
| | | | |
| 16 | 0.139 | --- | --- |
| 17 | 0.166 | --- | --- |
| 18 | 0.123 | --- | --- |
| 19 | 0.161 | --- | --- |
| 20 | 0.178 | --- | --- |

### Reference Example 6:

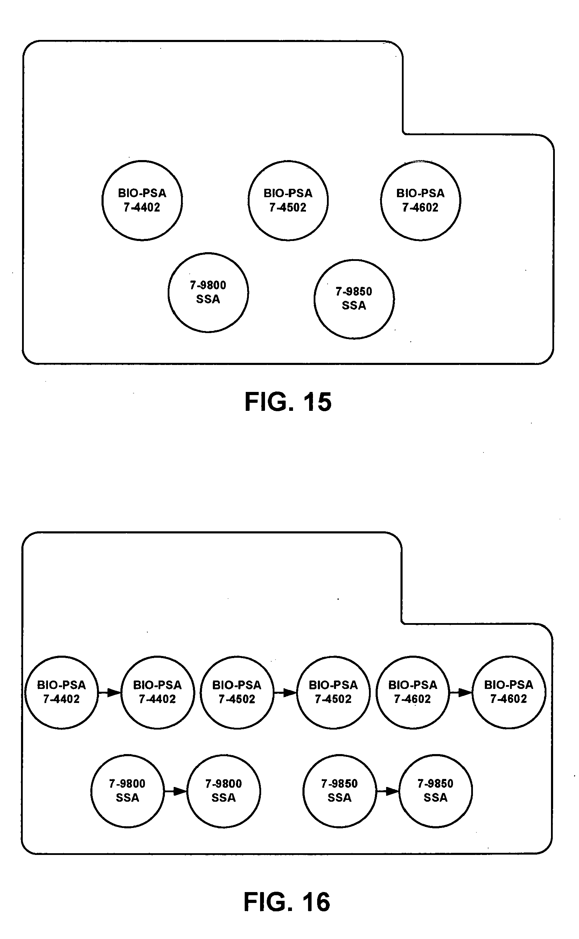

Two pacemakers (available from, for example, Medtronic, Inc., Minneapolis, MN) without a parylene coating were surface cleaned. Five PSA slabs were formed from different silicone PSAs: BIO-PSA 7-4402, BIO-PSA 7-4502, BIO-PSA 7-4602, 7-9800 SSA, and 7-9850 SSA, each of which is available from Dow Coming Corp, Midland, MI. The PSA slabs were applied to the front of the housing in the configuration shown in FIG. 15, and applied to the back of the housing in the configuration shown in FIG. 16. The PSA slabs applied to the back of the housing were removed once and repositioned as shown in FIG. 16. The BIO-PSA 7-4402 was relatively easy to peel off and reposition; however, it was difficult to grab the edge of the slab to begin peeling. BIO-PSA 7-4502 was more difficult to peel off the housing than BIO-PSA 7-4402. BIO-PSA 7-4602 was even more difficult to peel off the housing than BIO-PSA 7-4502. The 7-9800 SSA was gummy and very easy to peel off the housing, as was the 7-9850 SSA. All five of the PSA slabs adhered to the pacemaker housing on first application and held when the housing was turned upside down so the slabs were oriented toward the floor. Overall, the BIO-PSAs held better than the SSAs when scratched with a tweezers. All five PSA slabs could be peeled off and repositioned and still adhere to the housing.

### Reference Example 7:

Two pacemakers without a parylene coating were surface cleaned. Five PSA slabs were formed from different PSAs: BIO-PSA 7-4402, BIO-PSA 7-4502, BIO-PSA 7-4602, 7-9800 SSA, and 7-9850 SSA. The pacemaker was then wet with a room temperature aqueous solution including 0.9 wt. % NaCl (a 0.9% saline solution). The adhesive slabs were then applied according to the pattern and method described with respect to Example 6 and shown in FIGS. 15 and 16. The results followed the same general pattern as Example 6, as BIO-PSA 7-4402 was relatively easy to peel off, BIO-PSA 7-4502 was more difficult to peel off, BIO-PSA 7-4602 was the most difficult to peel off, and 7-9800 SSA, and 7-9850 SSA were the easiest to peel off. All five adhesive slabs adhered to the pacemaker when the pacemaker was turned to that the slabs were oriented towards the ground. The titanium housing of the pacemaker is very hydrophobic. The saline beaded up and rolled off the housing quickly, so it probably did not wet the pacemaker effective. After testing, both pacemakers were placed in a 0.9% saline solution and placed in a 37 °C orbital shaker to test longer term wet adhesion.

### Reference Example 8:

In this example, disks including a PSA, minocycline HCl and rifampin were prepared. Approximately 1.70 grams of a biocompatible PSA (available under the trade designation BIO-PSA 7-4602, from Dow Coming, Midland, MI) was added to approximately 0.75 grams of THF and approximately 0.82 grams Solutol® HS 15, available from BASF, Florham Park, N.J. The mixture was speed mixed until substantially homogeneous. The resulting mixture was placed in an oven at approximately 50 °C for approximately 1 hour to remove a majority of solvent. Approximately 0.60 grams of minocycline HCl and approximately 0.59 grams of rifampin were then added to the mixture and speed mixed for approximately 3 minutes at about 3500 RPM using a SpeedMixer™ DAC 150, available from FlackTek, Inc., Landrum, SC.

The resulting mixture, including the BIO-PSA 7-4602, rifampin and minocycline HCl was roll milled at ambient temperature using a variable speed roll mill (Rotomill Lab, available from Rondol Technology, Ltd., Stone, Staffordshire, United Kingdom), for about 20 minutes to about 30 minutes, or until the mixture was visually homogeneous. A portion of the mixture was placed between two release liners and pressed to a thickness of between approximately 0.025 inches and approximately 0.035 inches. Disks having a diameter of between approximately 0.75 inches and approximately 1.0 inch were die cut from the pressed film and evaluated for drug content and drug release characteristics. Each of the disks weighed between approximately 150 milligrams and approximately 200 milligrams. Drug content and drug release measurement were made using high performance liquid chromatography (HPLC). Drug release was measured in a phosphate buffered saline (PBS) solution maintained at approximately 37 °C and agitated at approximately 100 RPM in an orbital shaker (VWR® incubating Mini Shaker, VWR International, LLC, West Chester, PA).

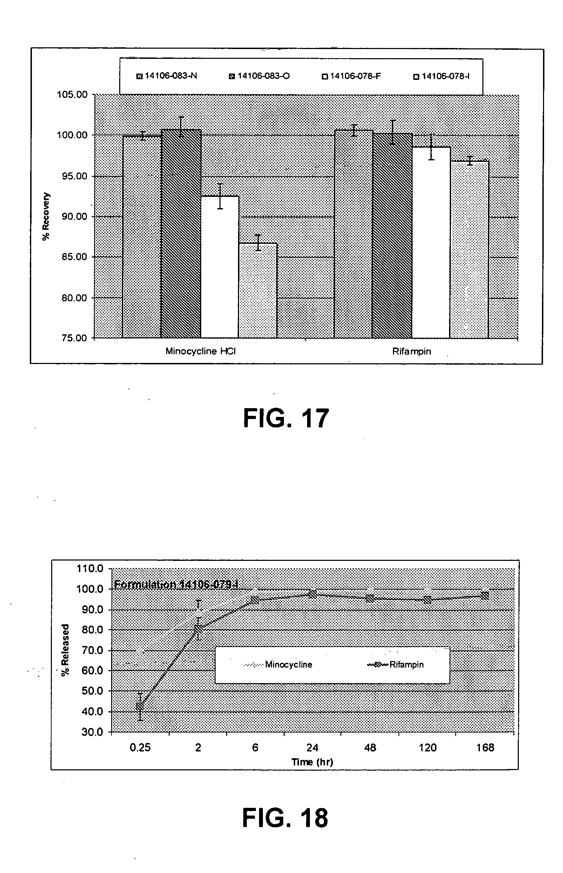

The percentage released of the total minocycline HCl and rifampin contained in the disk was determined from the drug content and release measurements. The results are shown in FIG. 17. Example 8 is labeled 14106-083-F. FIG. 17 shows error bars representing a 95% confidence interval for the data. The sample size was n=3 for the drug content assay and n=1 for the drug release data.

### Reference Example 9:

In this example, disks including a PSA, minocycline HCl and rifampin were prepared. Approximately 2.22 grams of 60 wt. % BIO-PSA 7-4602 in ethyl acetate, available from Dow Coming, Midland, MI, was mixed with approximately 1.72 grams of Kollidon® PF, available from BASF, Florham Park, N.J., and approximately 1.77 grams sucrose palmitate (available under the trade designation D1615, from Mitsubishi Tanabe Pharma America, Inc. Warren N.J.). The mixture was speed mixed at approximately 3500 RPM for about 3 minutes using a SpeedMixer™ DAC 150, available from FlackTek, Inc., Landrum, SC. The resulting mixture was placed on a release liner in an oven at approximately 70 °C for approximately 1 hour to remove a majority of solvent. Approximately 0.55 grams of minocycline HCl and approximately 0.53 grams of rifampin were then added to the mixture and speed mixed for approximately 3 minutes at about 3500 RPM using a SpeedMixer™ DAC 150, available from FlackTek, Inc., Landrum, SC.

The resulting mixture, including the BIO-PSA 7-4602, Kollidon® PF, sucrose palmitate, rifampin and minocycline HCl was roll milled at ambient temperature using a variable speed roll mill (Rotomill Lab, available from Rondol Technology, Ltd., Stone, Staffordshire, United Kingdom), for about 20 minutes to about 30 minutes, or until the mixture was visually homogeneous. A portion of the mixture was placed between two release liners and pressed to a thickness of between approximately 0.025 inches (0.635 mm) and approximately 0.035 inches (0.889 mm). Disks having a diameter of between approximately 0.75 inches (19.05 mm) and approximately 1.0 inch (25.4 mm) were die cut from the pressed film and evaluated for drug content and drug release characteristics. Each of the disks weighed between approximately 150 milligrams and approximately 200 milligrams. Drug content and drug release measurement were made using high performance liquid chromatography (HPLC). Drug release was measured in a phosphate buffered saline (PBS) solution maintained at approximately 37 °C and agitated at approximately 100 RPM in an orbital shaker (VWR® incubating Mini Shaker, VWR International, LLC, West Chester, PA).

The percentage released of the total minocycline HCl and rifampin contained in the disk was determined from the drug content and release measurements. The results are shown in FIG. 17. Example 9 is labeled 14106-079-I. FIG. 17 shows error bars representing a 95% confidence interval for the data. The sample size was n=3 for the drug content assay and n=1 for the drug release data. The elution profile is shown in FIG. 18 as percent minocycline HCl and rifampin released as a function of time. Within approximately 6 hours, substantially all the minocycline HCl was released, and approximately 95% of the rifampin was released.

### Reference Example 10:

In this example, disks including a PSA, minocycline HCl and rifampin were prepared. Approximately 8.12 grams of 60 wt. % BIO-PSA 7-4602 in ethyl acetate, available from Dow Coming, Midland, MI, was mixed with approximately 0.860 grams of Polysorbate-80 (polyoxyethylene (20) sorbitan monooleate), available from SAFC®, St. Louis, MO, and approximately 2.54 grams sucrose stearate (available under the trade designation D1807, from Mitsubishi Tanabe Pharma America, Inc. Warren N.J.). The mixture was speed mixed at approximately 3500 RPM for about 3 minutes using a SpeedMixer^{™} DAC 150, available from FlackTek, Inc., Landrum, SC. The resulting mixture was placed on a release liner in an oven at approximately 70 °C for approximately 1 hour to remove a majority of solvent. Approximately 0.99 grams of minocycline HCl and approximately 1.01 grams of rifampin were then added to the mixture and speed mixed for approximately 3 minutes at about 3500 RPM using a SpeedMixer™ DAC 150, available from FlackTek, Inc., Landrum, SC.

The resulting mixture, including the BIO-PSA 7-4602, Polysorbate-80, sucrose stearate, rifampin and minocycline HCl was roll milled at ambient temperature using a variable speed roll mill (Rotomill Lab, available from Rondol Technology, Ltd., Stone, Staffordshire, United Kingdom), for about 20 minutes to about 30 minutes, or until the mixture was visually homogeneous. A portion of the mixture was placed between two release liners and pressed to a thickness of between approximately 0.025 inches (0.635 mm) and approximately 0.035 inches (0.889 mm). Disks having a diameter of between approximately 0.75 inches (19.05 mm) and approximately 1.0 inch (25.4 mm) were die cut from the pressed film and evaluated for drug content and drug release characteristics. Each of the disks weighed between approximately 150 milligrams and approximately 200 milligrams. Drug content and drug release measurement were made using high performance liquid chromatography (HPLC). Drug release was measured in a phosphate buffered saline (PBS) solution maintained at approximately 37 °C and agitated at approximately 100 RPM in an orbital shaker (VWR® Incubating Mini Shaker, VWR International, LLC, West Chester, PA).

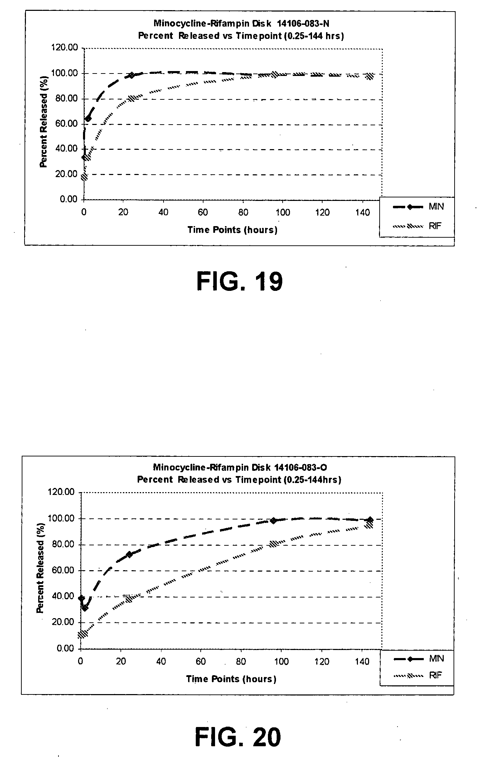

The percentage released of the total minocycline HCl and rifampin contained in the disk was determined from the drug content and release measurements. The results are shown in FIG. 17. Example 9 is labeled 14106-083-N. FIG. 17 shows error bars representing a 95% confidence interval for the data. The sample size was n=3 for the drug content assay and n=1 for the drug release data. The elution profile is shown in FIG. 19 as percent minocycline HCl and rifampin released as a function of time. Within approximately 24 hours, substantially all the minocycline HCl was released. Substantially all the rifampin was release within approximately 100 hours.

### Reference Example 11:

In this example, disks including a PSA, minocycline HCl and rifampin were prepared. Approximately 8.26 grams of 60 wt. % BIO-PSA 7-4602 in ethyl acetate was mixed with approximately 0.55 grams of Polysorbate-80, approximately 0.807 grams D1807, approximately 0.83 grams sucrose stearate (available under the trade designation D1803, from Mitsubishi Tanabe Pharma America, Inc. Warren N.J.), and approximately 1.15 grams of sucrose stearate (available under the trade designation D1816, from Mitsubishi Tanabe Pharma America, Inc. Warren N.J.). The mixture was speed mixed at approximately 3500 RPM for about 3 minutes using a SpeedMixer™ DAC 150, available from FlackTek, Inc., Landrum, SC. The resulting mixture was placed on a release liner in an oven at approximately 70 °C for approximately 1 hour to remove a majority of solvent. Approximately 0.96 grams of minocycline HCl and approximately 1.03 grams of rifampin were then added to the mixture and speed mixed for approximately 3 minutes at about 3500 RPM using a SpeedMixer^{™} DAC 150, available from FlackTek, Inc., Landrum, SC.

The resulting mixture, including the BIO-PSA 7-4602, Polysorbate-80, sucrose stearate, rifampin and minocycline HCl was roll milled at ambient temperature using a variable speed roll mill (Rotomill Lab, available from Rondol Technology, Ltd., Stone, Staffordshire, United Kingdom), for about 20 minutes to about 30 minutes, or until the mixture was visually homogeneous. A portion of the mixture was placed between two release liners and pressed to a thickness of between approximately 0.025 inches (0.635 mm) and approximately 0.035 inches (0.889 mm). Disks having a diameter of between approximately 0.75 inches (19.05 mm) and approximately 1.0 inch (25.4 mm) were die cut from the pressed film and evaluated for drug content and drug release characteristics. Each of the disks weighed between approximately 150 milligrams and approximately 200 milligrams. Drug content and drug release measurement were made using high performance liquid chromatography (HPLC). Drug release was measured in a phosphate buffered saline (PBS) solution maintained at approximately 37 °C and agitated at approximately 100 RPM in an orbital shaker (VWR® Incubating Mini Shaker, VWR International, LLC, West Chester, PA).

The percentage released of the total minocycline HCl and rifampin contained in the disk was determined from the drug content and release measurements. The results are shown in FIG. 17. Example 9 is labeled 14106-083-O. FIG. 17 shows error bars representing a 95% confidence interval for the data. The sample size was n=3 for the drug content assay and n=1 for the drug release data. The elution profile is shown in FIG. 20 as percent minocycline HCl and rifampin released as a function of time. Within approximately 100 hours, substantially all the minocycline HCl was released. Substantially all the rifampin was release within approximately 145 hours.

### Reference Examples 12-15:

In Examples 12-15, a solution of a silicone adhesive, available under the trade designation MED-1137, from NuSil Silicone Technology, Carpinteria, CA, was dissolved in heptanes to a concentration of approximately 50 wt. % silicone and approximately 50 wt. % heptanes. In examples including rifampin and minocycline HCl, the minocycline was weighed into a disposable cup, then the rifampin was weighed into the same cup. Once the silicone was fully dissolved in the heptane, the heptane/silicone solution was weighed into the disposable cup containing the rifampin and minocycline HCl. The mixture was mixed at approximately 3500 RPM for about 3 minutes using a SpeedMixer^{™} DAC 150, available from FlackTek, Inc., Landrum, SC. The resulting mixture was spread onto a release liner using a wet film applicator and dried at room temperature for approximately 48 hours in a fume hood. Tables 4-7 show the compositions of Examples 12-15.

**Table 4: Example 12**

| **Material** | **Desired Amount (g)** | **Desired Amount (wt. %)** | **Actual Amount (g)** | **Actual Amount (wt. %)** |
|---|---|---|---|---|
| Silicone | | 100 | | 100 |
| Minocycline HCl | | 0 | | 0 |
| Rifampin | | 0 | | 0 |
| Total | | 100 | | 100 |

**Table 5: Example 13**

| **Material** | **Desired Amount (g)** | **Desired Amount (wt. %)** | **Actual Amount (g)** | **Actual Amount (wt. %)** |
|---|---|---|---|---|
| Silicone | 9.00 | 94.7 | 9.012 | 94.83 |
| Minocycline HCl | 0.25 | 2.6 | 0.243 | 2.56 |
| Rifampin | 0.25 | 2.6 | 0.248 | 2.61 |
| Total | 9.50 | 100 | 9.503 | 100 |

**Table 6: Example 14**

| **Material** | **Desired** | **Desired** | **Actual** | **Actual** |
|---|---|---|---|---|
| | **Amount (g)** | **Amount (wt. %)** | **Amount (g)** | **Amount (wt. %)** |
| Silicone | 8.50 | 85.0 | 9.011 | 85.62 |
| Minocycline HCl | 0.75 | 7.5 | 7.61 | 7.23 |
| Rifampin | 0.75 | 7.5 | 7.53 | 7.15 |
| Total | 10.00 | 100 | 10.525 | 100 |

**Table 7: Example 15**

| **Material** | **Desired Amount (g)** | **Desired Amount (wt. %)** | **Actual Amount (g)** | **Actual Amount (wt. %)** |
|---|---|---|---|---|
| Silicone | 7.50 | 75.0 | 7.530 | 75.10 |
| Minocycline HCl | 1.25 | 12.5 | 1.245 | 12.42 |
| Rifampin | 1.25 | 12.5 | 1.252 | 12.49 |
| Total | 10.00 | 100 | 10.027 | 100 |

### Reference examples 16-18:

For Examples 16-18, an antimicrobial accessory was made using a two-part liquid silicone rubber, available under the trade designation Silastic® Q7-4850, from Dow Coming, Midland, MI. Minocycline HCl was weighed into a disposable plastic cup and then rifampin was weighed into the same cup. Part A of the liquid silicone rubber then was weighed into the cup containing the minocycline HCl and rifampin. Finally, part B of the liquid silicone rubber was mixed into the cup containing the part A, minocycline HCl, and rifampin. The mixture was mixed at approximately 3500 RPM for about 3 minutes using a SpeedMixer^{™} DAC 150, available from FlackTek, Inc., Landrum, SC, stirred once by hand, then mixed in the SpeedMixer^{™} DAC 150 for about 1 minute at about 3500 RPM. The silicone mixture including the minocycline HCl and rifampin was cured using a hot press at a temperature of approximately 120 °C and a force approximately 20,000 pounds for about 5 minutes. Tables 8-10 show the formulations for Examples 16-18.

**Table 8: Example 16**

| **Material** | **Desired Amount (g)** | **Desired Amount (wt. %)** | **Actual Amount (g)** | **Actual Amount (wt. %)** |
|---|---|---|---|---|
| Part A | 4.50 | 47.4 | 4.497 | 47.26 |
| Part B | 4.50 | 47.4 | 4.490 | 47.19 |
| Minocycline HCl | 0.25 | 2.6 | 0.261 | 2.74 |
| Rifampin | 0.25 | 2.6 | 0.267 | 2.81 |
| Total | 9.50 | 100 | 9.515 | 100 |

**Table 9: Example 17**

| **Material** | **Desired Amount (g)** | **Desired Amount (wt. %)** | **Actual Amount (g)** | **Actual Amount (wt. %)** |
|---|---|---|---|---|
| Part A | 4.25 | 42.5 | 4.265 | 42.48 |
| Part B | 4.25 | 42.5 | 4.258 | 42.41 |
| Minocycline HCl | 0.75 | 7.5 | 0.754 | 7.51 |
| Rifampin | 0.75 | 7.5 | 0.762 | 7.59 |
| Total | 10.00 | 100 | 10.039 | 100 |

**Table 10: Example 18**

| **Material** | **Desired Amount (g)** | **Desired Amount (wt. %)** | **Actual Amount (g)** | **Actual Amount (wt. %)** |
|---|---|---|---|---|
| Part A | 3.75 | 37.5 | 3.761 | 37.58 |
| Part B | 3.75 | 37.5 | 3.740 | 37.37 |
| Minocycline HCl | 1.25 | 12.5 | 1.254 | 12.53 |
| Rifampin | 1.25 | 12.5 | 1.253 | 12.52 |
| Total | 10.00 | 100 | 10.008 | 100 |

### Reference Example 19:

For Example 19, an antimicrobial accessory was made using a two-part liquid silicone rubber, available under the trade designation Silastic® Q7-4850, from Dow Coming, Midland, MI. Minocycline HCl was weighed into a disposable plastic cup. Part A of the liquid silicone rubber then was weighed into the cup containing the minocycline HCl. Finally, part B of the liquid silicone rubber was mixed into the cup containing the part A and minocycline HCl. The mixture was mixed at approximately 3500 RPM for about 3 minutes using a SpeedMixer^{™} DAC 150, available from FlackTek, Inc., Landrum, SC, stirred once by hand, then mixed in the SpeedMixer™ DAC 150 for about 1 minute at about 3500 RPM. The silicone mixture including the minocycline HCl was cured using a hot press at a temperature of approximately 120 °C and a force approximately 20,000 pounds for about 5 minutes. Table 11 shows the formulation for Example 19.

**Table 11: Example 19**

| **Material** | **Desired Amount (g)** | **Desired Amount (wt. %)** | **Actual Amount (g)** | **Actual Amount (wt. %)** |
|---|---|---|---|---|
| Part A | 4.38 | 43.8 | 4.360 | 43.68 |
| Part B | 4.38 | 43.8 | 4.370 | 43.75 |
| Minocycline HCl | 1.25 | 12.5 | 1.258 | 12.60 |
| Total | 10.00 | 100 | 9.988 | 100 |

### Reference Example 20:

For Example 20, an antimicrobial accessory was made using a two-part liquid silicone rubber, available under the trade designation Silastic® Q7-4850, from Dow Coming, Midland, MI, in a similar method as Example 19. However, rifampin was used instead of minocycline HCl. Table 12 shows the formulation for Example 20.

**Table 12: Example 20**

| **Material** | **Desired Amount (g)** | **Desired Amount (wt. %)** | **Actual Amount (g)** | **Actual Amount (wt. %)** |
|---|---|---|---|---|
| Part A | 4.38 | 43.8 | 4.388 | 43.81 |
| Part B | 4.38 | 43.8 | 4.381 | 43.74 |
| Rifampin | 1.25 | 12.5 | 1.248 | 12.46 |
| Total | 10.00 | 100 | 10.017 | 100 |

### Example 21:

In Example 21, an antimicrobial accessory was formed using a two-part liquid silicone rubber (available under the trade designation Silastic ® MDX4-4210 from Dow Coming Corp., Midland, MI). Minocycline HCl was weighed into a disposable plastic cup. Part A of the liquid silicone rubber then was weighed into the cup containing the rifampin. Finally, part B of the liquid silicone rubber was mixed into the cup containing the part A and minocycline HCl. The mixture was mixed at approximately 3500 RPM for about 5 minutes using a SpeedMixer™ DAC 150, available from FlackTek, Inc., Landrum, SC. The silicone mixture including the minocycline HCl was spread onto a release liner using the 40 millimeter end of a wet film applicator and dried for 48 hours at room temperature. Table 13 shows the formulation for Example 21.

**Table 13: Example 21**

| **Material** | **Desired Amount (g)** | **Desired Amount (wt. %)** | **Actual Amount (g)** | **Actual Amount (wt. %)** |
|---|---|---|---|---|
| Part A | 8.41 | 84.1 | 8.415 | 84.11 |
| Part B | 0.84 | 8.4 | 0.837 | 8.37 |
| Minocycline HCl | 0.75 | 7.5 | 0.753 | 7.53 |
| Total | 10.00 | 100 | 10.005 | 100 |

### Reference Example 22:

In Example 22, an antimicrobial accessory was formed using a two-part liquid silicone rubber (available under the trade designation Silastic ® MDX4-4210 from Dow Coming Corp., Midland, MI) in a similar method as Example 21, except rifampin was used instead of minocycline HCl. Table 14 shows the formulation for Example 22.

**Table 14: Example 22**

| **Material** | **Desired Amount (g)** | **Desired Amount (wt. %)** | **Actual Amount (g)** | **Actual Amount (wt. %)** |
|---|---|---|---|---|
| Part A | 8.41 | 84.1 | 8.406 | 84.04 |
| Part B | 0.84 | 8.4 | 0.838 | 8.38 |
| Rifampin | 0.75 | 7.5 | 0.758 | 7.58 |
| Total | 10.00 | 100 | 10.002 | 100 |

### Reference Examples 23-26:

In Examples 23-26, a solution of a silicone adhesive, available under the trade designation MED-1137, from NuSil Silicone Technology, Carpinteria, CA, was dissolved in heptane to a concentration of approximately 50 wt. % silicone and approximately 50 wt. % heptane. Example 23 included only silicone. Example 24 included rifampin and minocycline HCl: the minocycline was weighed into a disposable cup, then the rifampin was weighed into the same cup. Examples 25 and 26included only minocycline HCl and rifampin, respectively. In Example 25, the minocycline HCl was weighed into a disposable cup, while in Example 26, the rifampin was weighed into a disposable cup. Once the silicone was fully dissolved in the heptane, the heptane/silicone solution was weighed into the disposable cup containing the antimicrobials. The mixture was mixed at approximately 3500 RPM for about 3 minutes using a SpeedMixer™ DAC 150, available from FlackTek, Inc., Landrum, SC. The resulting mixture was spread onto a release liner using a wet film applicator and dried at room temperature for approximately 48 hours in a fume hood. Tables 15-18 show the compositions of Examples 23-26.

**Table 15: Example 23**

| **Material** | **Desired Amount (g)** | **Desired Amount (wt. %)** | **Actual Amount (g)** | **Actual Amount (wt. %)** |
|---|---|---|---|---|
| Silicone | | 100 | | 100 |
| Minocycline HCl | | 0 | | 0 |
| Rifampin | | 0 | | 0 |
| Total | | 100 | | 100 |

**Table 16: Example 24**

| **Material** | **Desired Amount (g)** | **Desired Amount (wt. %)** | **Actual Amount (g)** | **Actual Amount (wt. %)** |
|---|---|---|---|---|
| Silicone | 7.5 | 75.0 | 7.526 | 74.96 |
| Minocycline HCl | 1.25 | 12.5 | 1.258 | 12.53 |
| Rifampin | 1.25 | 12.5 | 1.256 | 12.51 |
| Total | 10.00 | 100 | 10.040 | 100 |

**Table 17: Example 25**

| **Material** | **Desired Amount (g)** | **Desired Amount (wt. %)** | **Actual Amount (g)** | **Actual Amount (wt. %)** |
|---|---|---|---|---|
| Silicone | 8.75 | 87.5 | 8.743 | 87.52 |
| Minocycline HCl | 1.25 | 12.5 | 1.258 | 12.58 |
| Total | 10.00 | 100 | 10.001 | 100 |

**Table 18: Example 26**

| **Material** | **Desired Amount (g)** | **Desired Amount (wt. %)** | **Actual Amount (g)** | **Actual Amount (wt. %)** |
|---|---|---|---|---|
| Silicone | 8.75 | 87.5 | 8.731 | 87.43 |
| Rifampin | 1.25 | 12.5 | 1.255 | 12.57 |
| Total | 10.00 | 100 | 9.986 | 100 |

### Reference Examples 27-29:

For Examples 27-29, an antimicrobial accessory was made using a two-part liquid silicone rubber, available under the trade designation Silastic® Q7-4850, from Dow Coming, Midland, MI. Example 27 included no antimicrobial, but included mannitol, which is an excipient that may increase an elution rate of antimicrobial in the antimicrobial accessory. Examples 28 included minocycline HCl, rifampin, and mannitol.

Parts A and B of the liquid silicone rubber were mixed at approximately 3500 RPM for about 5 minutes using a using a SpeedMixer™ DAC 150, available from FlackTek, Inc., Landrum, SC. The minocycline HCl, rifampin and/or mannitol was added to the parts A and B and mixed at approximately 3500 RPM for about 5 minutes using the SpeedMixer™ DAC 150. The mixture was then hand mixed with a spatula to remove material from the sides of the mixing cup, and mixed again SpeedMixer™ DAC 150 for about 5 minutes at approximately 3500 RPM. The mixtures were then molded in a LSR injection molding press in a thick book mold between two pieces of release liner at about 250 °C for about 5 minutes. Tables 19-21 show the compositions for Examples 27-29.

**Table 19: Example 27**

| **Material** | **Actual Amount (g)** | **Actual Amount (wt. %)** |
|---|---|---|
| Part A | 4.591 | 45.31 |
| Part B | 4.517 | 44.58 |
| Minocycline HCl | 0 | 0 |
| Rifampin | 0 | 0 |
| Mannitol | 1.025 | 10.11 |
| Total | 10.008 | 100 |

**Table 20: Example 28**

| **Material** | **Actual Amount (g)** | **Actual Amount (wt. %)** |
|---|---|---|
| Part A | 7.026 | 35.12 |
| Part B | 6.945 | 34.71 |
| Minocycline HCl | 2.002 | 10.01 |
| Rifampin | 2.010 | 10.05 |
| Mannitol | 2.023 | 10.11 |
| Total | 10.008 | 100 |

**Table 21: Example 29**

| **Material** | **Actual Amount (g)** | **Actual Amount (wt. %)** |
|---|---|---|
| Part A | 7.098 | 32.00 |
| Part B | 7.079 | 31.91 |
| Minocycline HCl | 2.003 | 9.03 |
| Rifampin | 2.012 | 9.07 |
| Mannitol | 3.990 | 17.99 |
| Total | 22.182 | 100 |

### Reference Examples 30-54:

Examples 30-54 illustrate zone of inhibition (ZOI) data collected for antimicrobial accessories including minocycline HCl and/or rifampin. Various polymers and excipients were used in combination with minocycline HCl and/or rifampin. Table 22 shows the formulations for each of examples 30-54 and the corresponding average ZOI. In Table 22, M stands for minocycline HCl, R stands for rifampin, SiO₂ is silica, PVP is polyvinylpyrrolidone, BIO-PSA 7-4601 is a silicone PSA available from Dow Coming Corp, Midland, MI, MDX4-4210 is a liquid silicone rubber available from Dow Coming, Corp., Midland, MI, LSR Q7-4850 is a liquid silicone rubber available from Dow Coming, Corp., Midland, MI, and MED-1137 a silicone adhesive available from NuSil Silicone Technology, Carpinteria, CA. All percentages listed in Table 22 are by weight.

**Table 22: Examples 30-54**

| **Sample** | **Polymer** | **Antimicrobial** | **Excipient** | **Average ZOI (mm)** |
|---|---|---|---|---|
| Example 30 | None | M | None | 16 |
| Example 31 | None | R | None | 17 |
| Example 32 | None | M and R | None | 17 |
| Example 33 | BIO-PSA 7-4601 | None | None | 0 |
| Example 34 | BIO-PSA 7-4601 | 7.5% M and R | 0.4% PVP, 0.08% SiO₂ | 17.7 |
| Example 35 | BIO-PSA 7-4601 | 10% M and R | 0.6% PVP, 0/01 % SiO₂ | 17.0 |
| Example 36 | BIO-PSA 7-4601 | 15% M and R | 0.6% PVP | 16.3 |
| Example 37 | BIO-PSA 7-4601 | 7.5% M | 0.6% PVP | 12.7 |
| Example 38 | BIO-PSA 7-4601 | 7.5% R | 0.6% PVP | 15.7 |
| Example 39 | MDX4-4210 | None | None | 5 |
| Example 40 | MDX4-4210 | 5% M and R | None | 16.3 |
| Example 41 | MDX4-4210 | 5% M and R | 10% glycerin | 17.3 |
| Example 42 | MDX4-4210 | 10% M and R | 5% glycerin | 17.3 |
| Example 43 | MDX4-4210 | 15% M and R | None | 18.3 |
| Example 44 | MDX4-4210 | 15% M and R | 10% glycerin | 17.0 |
| Example 45 | MDX4-4210 | 7.5% M | None | 17.0 |
| Example 46 | MDX4-4210 | 7.5% R | None | 18.7 |
| Example 47 | LSR Q7-4850 | None | None | 5 |
| Example 48 | LSR Q7-4850 | 25% M and R | None | 18.3 |
| Example 49 | LSR Q7-4850 | 12.5% M | None | 17.0 |
| Example 50 | LSR Q7-4850 | 12.5% R | None | 18.7 |
| Example 51 | MED-1137 | None | None | 6 |
| Example 52 | MED-1137 | 25% M and R | None | 17.7 |
| Example 53 | MED-1137 | 12.5% M | None | 15.0 |
| Example 54 | MED-1137 | 12.5% R | None | 17.7 |

### Reference Examples 55-58:

Examples 55-58 illustrate zone of inhibition (ZOI) data collected for antimicrobial accessories including minocycline HCl and rifampin. Various polymers and excipients were used in combination with minocycline HCl and/or rifampin. Example 55 included a silicone PSA available under the trade designation BIO-PSA 7-4601, from Dow Coming Corp, Midland, MI, minocycline HCl, rifampin, polyvinylpyrrolidone, and silicon dioxide. Example 56 included a LSR available under the trade designation MDX4-4210 from Dow Coming, Corp., Midland, MI, minocycline, rifampin, and glycerin. Example 57 included a LSR available under the trade designation Silastic® Q7-4850 from Dow Coming, Corp., Midland, MI, minocycline HCl, and rifampin. Example 58 included a silicone adhesive available under the trade designation MED-1137 from NuSil Silicone Technology, Carpinteria, CA, minocycline HCl, and rifampin. Table 23 shows the compositions of Examples 55-58. In Table 23, M stands for minocycline HCl, R stands for rifampin, SiO₂ is silica, and PVP is polyvinylpyrrolidone. All percentages listed in Table 23 are weight percentages.

**Table 23: Examples 55-58**

| **Sample** | **Polymer** | **Antimicrobial** | **Excipient** |
|---|---|---|---|
| Example 55 | BIO-PSA 7-4601 | 3.75% M; 3.75% R | 0.4% PVP, 0.08% SiO₂ |
| Example 56 | MDX4-4210 | 5% M; 5% R | 5% glycerin |
| Example 57 | LSR Q7-4850 | 12.5% M; 12.5% R | None |
| Example 58 | MED-1137 | 12.5% M; 12.5% R | None |

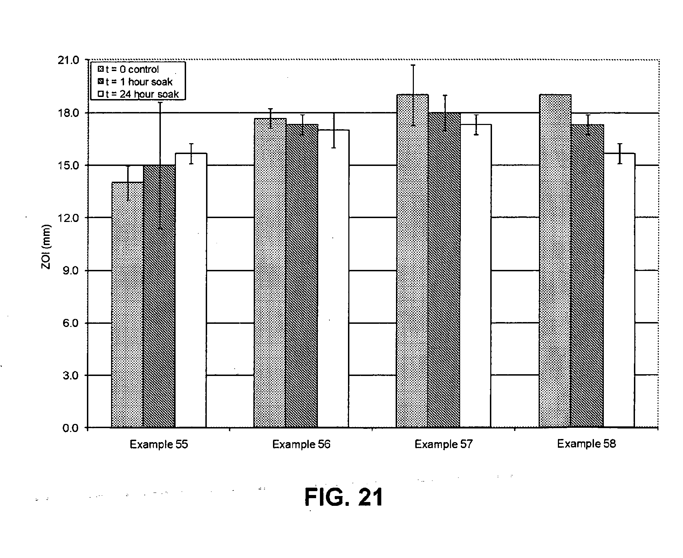

The resulting ZOI data is shown in FIG. 21. Three samples of each Example were made and tested. The ZOI was measured with a ruler after soaking of the antimicrobial accessory for 1 hour and for 24 hours, and a control measurement was made when the antimicrobial accessory was initially placed in the test solution. A 95% confidence interval was calculated based on the results of the tree samples for each Example and is also shown in FIG. 21.

### Reference Examples 59-66:

Eight formulations of rifampin and minocycline HCl in various polymers were evaluated by assay, related substance and elution tests. Table 24 shown the compositions of the eight formulations. In Table 24, LSR is a liquid silicone rubber available under the trade designation Silastic® Q7-4850 from Dow Coming, Corp., Midland, MI; G is glycerin; M is minocycline HCl: R is rifampin; PVP is polyvinylpyrrolidone; PSA is a pressure sensitive adhesive; PEG 1000 is poly(ethylene glycol) with a molecular weight of 1000 g/mol; and MA is mannitol. All percentages listed are weight percentages. The layers including the antimicroibals in Examples 61 and 62 were laminated to an adhesive layer comprising BIO-PSA 7-4602, available from Dow Coming Corp., Midland MI.

**Table 24: Examples 59-66**

| **Sample** | **Name** | **Composition** |
|---|---|---|
| Example 59 | 51-4 | 58.7% LSR; 5.49% M; 7.83% R; 10% G; 18% NaCl |
| Example 60 | 49D | 16% PSA; 5.54% M; 6.58% R; 22% G; 50% PVP |
| Example 61 | 49-3 | 28% G; 60% PVP; 3.63% M; 6.86% R |
| Example 62 | 49-3E | 28% PEG 1000; 68% PVP; 1.09% M; 1.30% R |
| Example 63 | 49-3F | 18.8% G; 40% PVP; 35% PSA; 2.37% M; 4.49% R |
| Example 64 | 14257-16-5 | 80% LSR; 10%M; 10% R |
| Example 65 | 14257-20-1 | 70% LSR; 10% M; 10% R; 10% MA |
| Example 66 | 14257-23-1 | 70% LSR; 10% M; 10% R; 10% G |

Assay results ranged from 52.4-104.5% for minocycline HCl and 53.6-102.3% for rifampin. These ranges indicate what percent of the theoretical total antimicrobial content was recovered from the samples when tested. Related substance analysis demonstrated that epi-minocycline and rifampin quinone were the two principle degradation products. Addition of these two peaks to the percent recovery of the respective parent (i.e., minocycline HCl and rifampin) peak resulted in total recovery of the respective antimicrobial between approximately 80% and approximately 120%, with the exception of formulations 51-4 and 14257-23-1, both of which contain glycerin and LSR. The two remaining LSR samples (14257-16-5 and 14257-20-1) demonstrated greatest recovery and lowest degradation of the samples tested.

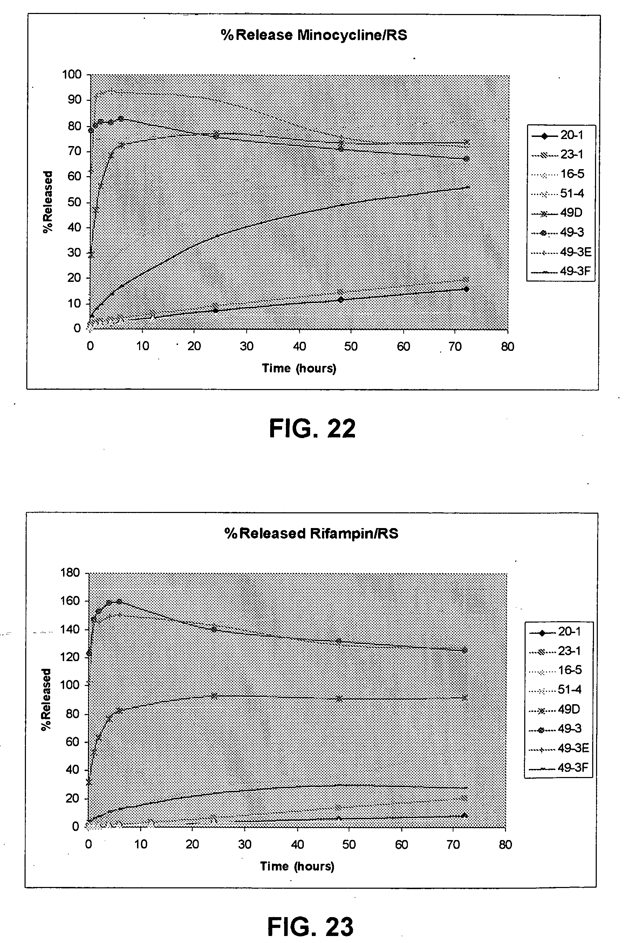

For elution testing, disk-shaped antimicrobial accessories including the various compositions were placed in simulated body fluid (phosphate buffered saline) and shaken gently at approximately 37 °C. After 1 hour, 2 hours, 4 hours, 6 hours, 12 hours, 24 hours, 72 hours, 120 hours, and 168 hours, 3 disks were removed from the simulated body fluid and the remaining drug was extracted by shaking in organic solvent. Remaining antimicrobial content was measured using HPLC. The remaining antimicrobial content for each sample was compared to initial drug content from antimicrobial accessory control samples. Results from the elution analysis demonstrated significant degradation of both minocycline HCl and rifampin. As discussed above, epi-minocycline and rifampin quinone were the major degradation products. To account for this degradation, the results of each of the degradation products were added back to the corresponding parent peak. Results for the minocycline HCl and rifampin elution are presented in FIGS. 22 and 23, respectively.

FIGS. 22 and 23 illustrate that the non-LSR formulations generally showed quicker initial elution of minocycline HCL and rifampin as well as more complete elution following 72 hours. Although the addition of glycerin or glycerin and sodium chloride did speed the elution rate and extent up for the LSR formulations, elution was slower and less complete from the LSR formulations than from non-LSR formulations, with the exception of 49-3F, which eluted similar to the LSR samples.

### Reference Examples 67-69:

An in-vivo study of antimicrobial efficacy of an antimicrobial accessory used with a single chamber Medtronic pacemaker with a cut silicone lead was performed. Devices and leads were implanted subcutaneously into rabbits, and S. aureus was injected into the device pocket at the time of implantation. Three quarter inch diameter disks of the formulations listed in Table 25 were implanted with the pacemaker in test group animals. No antimicrobial accessories were implanted in control animals. After 7 days the pacemakers, leads, antimicrobial accessory, subcutaneous pocket, and blood were tested for presence of S. aureus. A one hundred percent reduction in number of occurrences of the presence of the bacteria was observed in the test animals (0 out of 8 with infection) for all three antimicrobial disk formulations relative to control animals (7 out of 8 with infection). In Table 25, LSR stands for a liquid silicone rubber available under the trade designation Silastic® Q-7-4850 LSR, from Dow Coming, Corp., Midland, MI; M stands for minocycline HCl, R stand for rifampin, MA stands for mannitol, G stands for glycerin, and PVP stands for polyvinylpyrrolidone.

**Table 25: Examples 67-69**

| **Sample** | **Composition** |
|---|---|
| Example 67 | 70% LSR; 10% M; 10% R; 10% MA |
| Example 68 | 5% M; 5% R; 25% G; 60% PVP; 5% MA |
| Example 69 | 56% LSR; 6% M; 8% R; 10% G; 16% NaCl; 4% MA |

### Reference Examples 70-75:

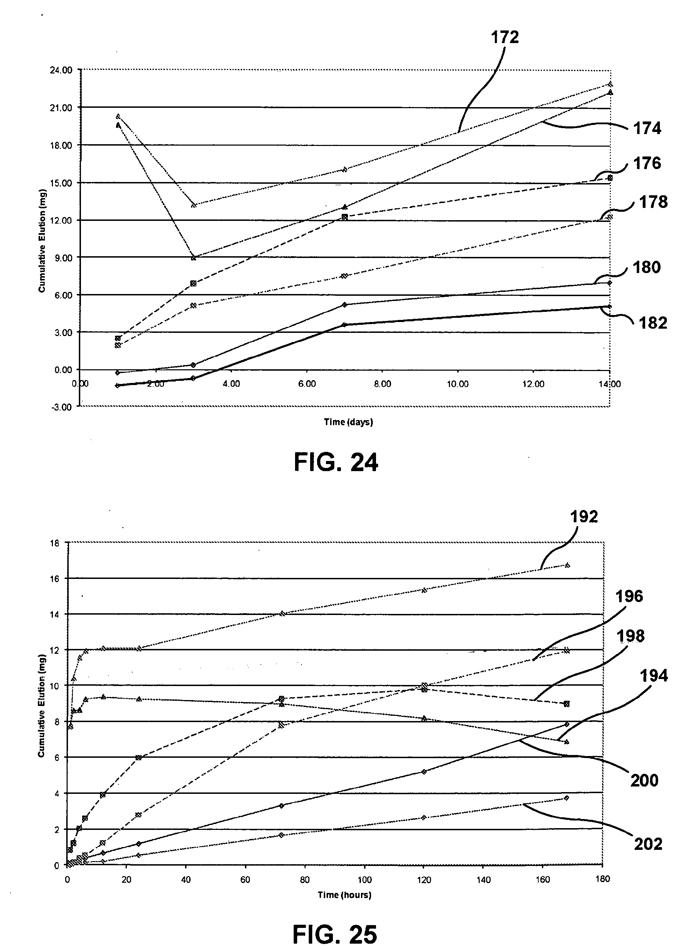

Samples including compositions describe above in Table 25 were subjected to in-vivo and in-vitro elution testing. For in-vivo elution testing, disk-shaped antimicrobial accessories including the various compositions were implanted subcutaneously in New Zealand White Rabbits. After 1 day, 3 days, 7 days, and 14 days, 3 rabbits were sacrificed and the implanted disk was explanted from each of the sacrificed rabbits. The antimicrobial content remaining in each of the disks was measured using high performance liquid chromatography (HPLC). The remaining antimicrobial content for each sample was compared to initial drug content from antimicrobial accessory control samples. FIG. 24 shows the results from the in vivo testing. Curve 172 illustrates the data collected for rifampin elution from a disk having a composition similar to Example 68. Curve 174 illustrates the data collected for minocycline HCL elution from a disk having a composition similar to Example 68. Curve 176 illustrates the data collected for minocycline HCL elution from a disk having a composition similar to Example 69. Curve 178 illustrates the data collected for rifampin elution from a disk having a composition similar to Example 69. Curve 180 illustrates the data collected for minocycline HCl elution from a disk having a composition similar to Example 67. Curve 182 illustrates the data collected for rifampin elution from a disk having a composition similar to Example 67.

For in-vitro elution testing, disk-shaped antimicrobial accessories including the various compositions were placed in simulated body fluid (phosphate buffered saline) and shaken gently at approximately 37 °C. After 1 hour, 2 hours, 4 hours, 6 hours, 12 hours, 24 hours, 72 hours, 120 hours, and 168 hours, 3 disks were removed from the simulated body fluid and the remaining drug was extracted by shaking in organic solvent. Remaining antimicrobial content was measured using HPLC. The remaining antimicrobial content for each sample was compared to initial drug content from antimicrobial accessory control samples. FIG. 25 shows the results from the in-vitro testing. Curve 192 illustrates the data collected for rifampin elution from a disk having a composition similar to Example 68. Curve 194 illustrates the data collected for minocycline HCl elution from a disk having a composition similar to Example 68. Curve 196 illustrates the data collected for rifampin elution from a disk having a composition similar to Example 69. Curve 198 illustrates the data collected for minocycline HCl elution from a disk having a composition similar to Example 69. Curve 200 illustrates the data collected for minocycline HCl elution from a disk having a composition similar to Example 67. Curve 202 illustrates the data collected for rifampin elution from a disk having a composition similar to Example 67.

## Claims

1. A system comprising:
an implantable medical device (16) comprising a housing (40); and
an antimicrobial accessory (26) comprising:
at least one first domain comprising a pressure sensitive adhesive (52) and a first antimicrobial mixed in the pressure sensitive adhesive, and
at least one second domain comprising a second polymer and a second antimicrobial mixed in the second polymer; wherein the at least one first domain and the at least one second domain comprise different compositions; and
wherein the antimicrobial accessory is adhered to the housing by the at least one first domain.

2. The system of claim 1, wherein the first antimicrobial is different from the second antimicrobial.

3. The system of claim 1 or 2, wherein at least one of the first antimicrobial and the second antimicrobial comprises at least one of an antibiotic, an antiseptic, an antimicrobial peptide, or a quaternary ammonium.

4. The system of claim 1 or 2, wherein at least one of the first antimicrobial and the second antimicrobial comprises at least one of a tetracycline, a rifamycin, a macrolide, a penicillin, a cephalosporin, an aminoglycoside, a glycopeptides, a quinolone, fusidic acid, trimethoprim, metronidazole, mupirocin, a polene, an azole, a beta-lactam inhibitor, bacitracin, neomycin, tigecycline, daptomycin, or clindamycin.

5. The system of any of claims 1 to 4, wherein the pressure sensitive adhesive comprises at least one of a silicone pressure sensitive adhesive, a polyurethane pressure sensitive adhesive, an acrylic pressure sensitive adhesive, a cyanoacrylate pressure sensitive adhesive, a poly(lactic-*co*-glycolic acid)-based pressure sensitive adhesive, or a polyisobutylene pressure sensitive adhesive.

6. The system of any of claims 1 to 5, wherein the second polymer comprises at least one of silicone, polyurethane, collagen, poly(lactic-*co*-glycolic acid), poly(lactic acid), poly(glycolic acid), poly(ethylene oxide), poly(ortho ester), poly(ε-caprolactone), poly(dioxanone), polyglyconate, hyaluronic acid, gelatin, fibrin, fibrinogen, cellulose, starch, cellulose acetate, polyvinylpyrrolidone, a poly(ethylene oxide)/poly(propylene oxide) copolymer, poly(ethylene vinyl acetate), poly(hydroxybutyrate-covalerate), polyanhydride, poly(glycolic acid-*co*-trimethylene carbonate), a polyphosphoester, a polyphosphoester urethane, a poly(amino acid), a cyanoacrylate, poly(trimethylene carbonate), poly(iminocarbonate), a copoly(ether-ester), a polyalkylene oxalate, a polyphasphazene, a polyarylate, a polyacrylate, poly(vinyl alcohol), poly(vinyl acetate), carboxymethyl cellulose, poly(acrylic acid), a tyrosine-based biodegradable polymer, polyhydroxyalkanoate, or a sugar ester.

7. The system of any of claims 1 to 6, wherein the antimicrobial accessory further comprises an antioxidant, wherein the antioxidant comprises at least one of butylated hydroxyl toluene, vitamin E, vitamin A, or vitamin C, and wherein the antioxidant is mixed into at least one of the pressure sensitive adhesive or the second polymer.

8. The system of any of claims 1 to 7, wherein the antimicrobial accessory further comprises an excipient, wherein the excipient comprises at least one of poly(vinylpyrrolidone), polysorbate 80, polysorbate 20, sucrose stearate, stearyl alcohol, glycerol monostearate, mannitol, poly(ethylene glycol), or macrogol 15 hydroxystearate (IV), and wherein the excipient is mixed into at least one of the pressure sensitive adhesive or the second polymer.

9. A method of making a system as claimed in any of claims 1 to 8, said method comprising:
forming a first mixture of a first domain composition comprising a pressure sensitive adhesive (52) and a first antimicrobial mixed in the pressure sensitive adhesive;
forming a second mixture of a second domain composition comprising a second polymer and a second antimicrobial mixed in the second polymer, wherein the first mixture and the second mixture comprise different compositions;
forming a patterned antimicrobial accessory.

10. The method of claim 9, further comprising:
adhering the antimicrobial accessory to a housing of an implantable medical device using the pressure sensitive adhesive.

11. The method of claim 9 or 10, wherein the second polymer comprises at least one of silicone, polyurethane, collagen, poly(lactic-*co*-glycolic acid), poly(lactic acid), poly(glycolic acid), poly(ethylene oxide), poly(ortho ester), poly(ε-caprolactone), poly(dioxanone), polyglyconate, hyaluronic acid, gelatin, fibrin, fibrinogen, cellulose, starch, cellulose acetate, polyvinylpyrrolidone, a poly(ethylene oxide)/poly(propylene oxide) copolymer, poly(ethylene vinyl acetate), poly(hydroxybutyrate-covalerate), polyanhydride, poly(glycolic acid-*co*-trimethylene carbonate), a polyphosphoester, a polyphosphoester urethane, a poly(amino acid), a cyanoacrylate, poly(trimethylene carbonate), poly(iminocarbonate), a copoly(ether-ester), a polyalkylene oxalate, a polyphasphazene, a polyarylate, a polyacrylate, poly(vinyl alcohol), poly(vinyl acetate), carboxymethyl cellulose, poly(acrylic acid), a tyrosine-based biodegradable polymer, polyhydroxyalkanoate, or a sugar ester.

12. The method of claim 9 or 10, wherein the pressure sensitive adhesive comprises at least one of a silicone pressure sensitive adhesive, a polyurethane pressure sensitive adhesive, an acrylic pressure sensitive adhesive, a cyanoacrylate pressure sensitive adhesive, a poly(lactic-*co*-glycolic acid)-based pressure sensitive adhesive, or a polyisobutylene pressure sensitive adhesive.

13. The method of any of claims 9 to 12, further comprising sterilizing the antimicrobial accessory using at least one of electron-beam sterilization, gamma beam sterilization, ethylene oxide sterilization, or autoclaving.

## Patentansprüche

1. System, das enthält:
eine implantierbare medizinische Vorrichtung (16), die ein Gehäuse (40) aufweist; und
ein antimikrobielles Zubehör (26), das enthält:
wenigstens einen ersten Bereich, der einen druckempfindlichen Klebstoff (52) und einen in den druckempfindlichen Klebstoff gemischten ersten antimikrobiellen Wirkstoff enthält, und
wenigstens einen zweiten Bereich, der ein zweites Polymer und einen in das zweite Polymer gemischten antimikrobiellen Wirkstoff enthält; wobei der wenigstens eine erste Bereich und der wenigstens eine zweite Bereich verschiedene Zusammensetzungen aufweisen; und
wobei das antimikrobielle Zubehör durch den wenigstens einen ersten Bereich an dem Gehäuse haftet.

2. System nach Anspruch 1, wobei der erste antimikrobielle Wirkstoff von dem zweiten antimikrobiellen Wirkstoff verschieden ist.

3. System nach Anspruch 1 oder 2, wobei der erste antimikrobielle Wirkstoff und/oder der zweite antimikrobielle Wirkstoff ein Antibiotikum und/oder ein Antiseptikum und/oder ein antimikrobielles Peptid und/oder quaternäres Ammonium enthält.

4. System nach Anspruch 1 oder 2, wobei der erste antimikrobielle Wirkstoff und/oder der zweite antimikrobielle Wirkstoff Tetracyclin und/oder Rifamycin und/oder Makrolid und/oder Penicillin und/oder Cephalosporin und/oder Aminoglycosid und/oder Glycopeptid und/oder Quinilon und/oder Fusidinsäure und/oder Trimethoprin und/oder Metronidazol und/oder Mupirozin und/oder Polen und/oder Azol und/oder einen Betalactam-Inhibitor und/oder Bacitrazin und/oder Neomycin und/oder Tigecyclin und/oder Daptomycin und/oder Clindamycin enthält.

5. System nach einem der Ansprüche 1 bis 4, wobei der druckempfindliche Klebstoff einen druckempfindlichen Silikonklebstoff und/oder einen druckempfindlichen Polyurethan-Klebstoff und/oder einen druckempfindlichen Acryl-Klebstoff und/oder einen druckempfindlichen Cyanoacryl-Klebstoff und/oder einen druckempfindlichen Klebstoff auf Poly(Lactid-Co-Glycolsäure)-Basis und/oder einen druckemfindlichen Polyisobutylen-Klebstoff enthält.

6. System nach einem der Ansprüche 1 bis 5, wobei das zweite Polymer wenigstens eines der Folgenden enthält: Silikon, Polyurethan, Kollagen, Poly(Lactid-Co-Glycolsäure), Poly(Milchsäure), Poly(Glycolsäure), Poly(Ethylenoxid), Poly(Orthoester), Poly(ε-Kaprolacton), Poly(Dioxanon), Polyglyconat, Hyaluronsäure, Gelatine, Fibrin, Fibrinogen, Zellulose, Stärke, Zelluloseacetat, Polyvinylpyrrolidon, Poly(Ethylenoxid)/Poly(Propylen-oxid)-Copolymer, Poly(Ethylenvinylacetat), Poly(Hydroxybutyrat-Covalerat), Polyanhydrid, Poly(Glycolsäure-Co-Trimethylencarbonat), Polyphosphorester, Polyphosphorester-Urethan, Poly(Aminosäure), Cyanoacrylat, Poly(Trimethylen-Carbonat), Poly(Iminocarbonat), Copoly(Ester-Ester), Polyalkylen-Oxalat, Polyphasphazen, Polyarylat, Polyacrylat, Poly(Vinylalkohol), Poly(Vinylacetat, Carboxymethyl-Zellulose, Poly(Acrylsäure), bioabbaubares Polymer auf Tyrosin-Basis, Polyhydroxyalkanoat, Zucker-Ester.

7. System nach einem der Ansprüche 1 bis 6, wobei das antimikrobielle Zubehör ferner ein Antioxidans enthält, wobei das Antioxidans Butyl-Hydroxyl-Toluen und/oder Vitamin E und/oder Vitamin A und/oder Vitamin C enthält und wobei das Antioxidans in den druckempfindlichen Klebstoff und/oder in das zweite Polymer gemischt ist.

8. System nach einem der Ansprüche 1 bis 7, wobei das antimikrobielle Zubehör ferner einen Trägerstoff enthält, wobei der Trägerstoff wenigstens eines der Folgenden enthält: Poly(Vinylpyrrolidon), Polysorbat 80, Polysorbat 20, Sucrosestearat, Stearylalkohol, Glycerolmonostearat, Mannitol, Poly(Ethylenglycol) oder Makrogol-16-Hydroxystearat (IV), und wobei der Trägerstoff in den druckempfindlichen Klebstoff und/oder in das zweite Polymer gemischt ist.

9. Verfahren zum Herstellen eines Systems nach einem der Ansprüche 1 bis 8, wobei das Verfahren umfasst:
Bilden eines ersten Gemisches aus einer Zusammensetzung eines ersten Bereichs, die einen druckempfindlichen Klebstoff (52) und einen in den druckempfindlichen Klebstoff gemischten ersten antimikrobiellen Wirkstoff enthält;
Bilden eines zweiten Gemisches aus einer Zusammensetzung eines zweiten Bereichs, die ein zweites Polymer und einen in das zweite Polymer gemischten zweiten antimikrobiellen Wirkstoff enthält, wobei das erste Gemisch und das zweite Gemisch verschiedene Zusammensetzungen enthalten;
Bilden eines bemusterten antimikrobiellen Zubehörs.

10. Verfahren nach Anspruch 9, das ferner umfasst:
Befestigen durch Haftung des antimikrobiellen Zubehörs an einem Gehäuse einer implantierbaren medizinischen Vorrichtung unter Verwendung des druckempfindlichen Klebstoffs.

11. Verfahren nach Anspruch 9 oder 10, wobei das zweite Polymer eines der Folgenden enthält: Silikon, Polyurethan, Kollagen, Poly(Lactid-Co-Glycolsäure), Poly(Milchsäure), Poly(Glycolsäure), Poly(Ethylenoxid), Poly(Orthoester), Poly(ε-Kaprolacton), Poly(Dioxanon), Polyglyconat, Hyaluronsäure, Gelatine, Fibrin, Fibrinogen, Zellulose, Stärke, Zelluloseacetat, Polyvinylpyrrolidon, Poly(Ethylenoxid)/Poly(Propylen-oxid)-Copolymer, Poly(Ethylenvinylacetat), Poly(Hydroxybutyrat-Covalerat), Polyanhydrid, Poly(Glycolsäure-Co-Trimethylencarbonat), Polyphosphorester, Polyphosphorester-Urethan, Poly(Aminosäure), Cyanoacrylat, Poly(Trimethylen-Carbonat), Poly(Iminocarbonat), Copoly(Ester-Ester), Polyalkylen-Oxalat, Polyphasphazen, Polyarylat, Polyacrylat, Poly(Vinylalkohol), Poly(Vinylacetat, Carboxymethyl-Zellulose, Poly(Acrylsäure), bioabbaubares Polymer auf Tyrosin-Basis, Polyhydroxyalkanoat, Zucker-Ester.

12. Verfahren nach Anspruch 9 oder 10, wobei der druckempfindliche Klebstoff einen druckempfindlichen Silikonklebstoff und/oder einen druckempfindlichen Polyurethan-Klebstoff und/oder einen druckempfindlichen Acryl-Klebstoff und/oder einen druckempfindlichen Cyanoacryl-Klebstoff und/oder einen druckempfindlichen Klebstoff auf Poly(Lactid-Co-Glycolsäure)-Basis und/oder einen druckemfindlichen Polyisobutylen-Klebstoff enthält.

13. Verfahren nach einem der Ansprüche 9 bis 12, das ferner das Sterilisieren des antimikrobiellen Zubehörs unter Verwendung einer Elektronenstrahl-Sterilisation und/oder einer Gammastrahl-Sterilisation und/oder einer Ethylenoxid-Sterilisation und/oder einer Autoklaven-Sterilisation umfasst.

## Revendications

1. Système comportant :
un dispositif médical implantable (16) comportant un boîtier (40) ; et
un accessoire antimicrobien (26) comportant :
au moins un premier domaine comportant un adhésif sensible à la pression (52) et un premier antimicrobien mélangé dans l'adhésif sensible à la pression, et
au moins un second domaine comportant un second polymère et
un second antimicrobien mélangé dans le second polymère ;
dans lequel le au moins un premier domaine et le au moins un second domaine comportent différentes compositions ; et
dans lequel l'accessoire antimicrobien adhère au boîtier par le au moins un premier domaine.

2. Système selon la revendication 1, dans lequel le premier antimicrobien est différent du second antimicrobien.

3. Système selon la revendication 1 ou 2, dans lequel au moins un parmi le premier antimicrobien et le second antimicrobien comporte au moins un élément parmi un antibiotique, un antiseptique, un peptide antimicrobien ou un ammonium quaternaire.

4. Système selon la revendication 1 ou 2, dans lequel au moins un parmi le premier antimicrobien et le second antimicrobien comporte au moins un élément parmi une tétracycline, une rifamycine, un macrolide, une pénicilline, une céphalosporine, un aminoglycoside, un glycopeptide, une quinolone, de l'acide fusidique, du triméthoprime, du métronidazole, de la mupirocine, un pollen, un azole, un inhibiteur de bêta-lactame, la bacitracine, la néomycine, la tigécycline, la daptomycine ou la clindamycine.

5. Système selon l'une quelconque des revendications 1 à 4, dans lequel l'adhésif sensible à la pression comporte au moins un élément parmi un adhésif au silicone sensible à la pression, un adhésif au polyuréthanne sensible à la pression, un adhésif acrylique sensible à la pression, un adhésif au cyanoacrylate sensible à la pression, un adhésif à base d'acide poly(lactique-co-glycolique) sensible à la pression, ou un adhésif au polyisobutylène sensible à la pression.

6. Système selon l'une quelconque des revendications 1 à 5, dans lequel le second polymère comporte au moins un élément parmi : silicone, polyuréthanne, collagène, acide poly(lactique-co-glycolique), acide poly(lactique), acide poly(glycolique), oxyde de poly(éthylène), poly(ortho-ester), poly(ε-caprolactone), poly(dioxanone), polyglyconate, acide hyaluronique, gélatine, fibrine, fibrinogène, cellulose, amidon, acétate de cellulose, polyvinylpyrrolidone, copolymère d'oxyde de poly(éthylène)/poly(propylène), poly(éthylène-acétate de vinyle), poly(hydroxybutyrate-covalérate), polyanhydride, poly(acide glycolique-co-triméthylène carbonate), polyphosphoester, polyphosphoester uréthane, poly(amino-acide), cyanoacrylate, poly(triméthylène carbonate), poly(iminocarbonate), copoly(éther-ester), oxalate de polyalkylène, polyphasphazène, polyarylate, polyacrylate, alcool poly(vinylique), poly(acétate de vinyle), carboxyméthyl cellulose, acide poly(acrylique), polymère biodégradable à base de tyrosine, polyhydroxyalcanoate ou ester de sucre.

7. Système selon l'une quelconque des revendications 1 à 6, dans lequel l'accessoire antimicrobien comporte en outre un antioxydant, dans lequel l'antioxydant comporte au moins un élément parmi l'hydroxytoluène butylé, la vitamine E, la vitamine A ou la vitamine C, et dans lequel l'antioxydant est mélangé dans au moins un élément parmi d'adhésif sensible à la pression ou le second polymère.

8. Système selon l'une quelconque des revendications 1 à 7, dans lequel l'accessoire antimicrobien comporte en outre un excipient, dans lequel l'excipient comporte au moins un élément parmi poly(vinylpyrrolidone), polysorbate 80, polysorbate 20, stéarate de saccharose, alcool stéarylique, monostéarate de glycérol, mannitol, poly(éthylène glycol) ou macrogol 15 hydroxystéarate (IV), et dans lequel l'excipient est mélangé dans au moins un élément parmi l'adhésif sensible à la pression ou le second polymère.

9. Procédé de fabrication d'un système tel que revendiqué dans l'une quelconque des revendications 1 à 8, ledit procédé comportant les étapes consistant à :
former un premier mélange d'une composition de premier domaine comportant un adhésif sensible à la pression (52) et
un premier antimicrobien mélangé dans l'adhésif sensible à la pression ;
former un second mélange d'une composition de second domaine comportant un second polymère et un second antimicrobien mélangé dans le second polymère, dans lequel le premier mélange et le second mélange comportent différentes compositions ;
former un accessoire antimicrobien muni d'un motif.

10. Procédé selon la revendication 9, comportant en outre l'étape consistant à :
faire adhérer l'accessoire antimicrobien à un boîtier d'un dispositif médical implantable en utilisant l'adhésif sensible à la pression.

11. Procédé selon la revendication 9 ou 10, dans lequel le second polymère comporte au moins un élément parmi : silicone, polyuréthanne, collagène, acide poly(lactique-coglycolique), acide poly(lactique), acide poly(glycolique), oxyde de poly(éthylène), poly(ortho-ester), poly(ε-caprolactone), poly(dioxanone), polyglyconate, acide hyaluronique, gélatine, fibrine, fibrinogène, cellulose, amidon, acétate de cellulose, polyvinylpyrrolidone, copolymère d'oxyde de poly(éthylène)/poly(propylène), poly(éthylène-acétate de vinyle), poly(hydroxybutyrate-covalérate), poly-anhydride, poly(acide glycolique-co-triméthylène carbonate), polyphosphoester, polyphosphoester uréthane, poly(amino-acide), cyanoacrylate, poly(triméthylène carbonate), poly(iminocarbonate), copoly(éther-ester), oxalate de polyalkylène, polyphasphazène, polyarylate, polyacrylate, alcool poly(vinylique), poly(acétate de vinyle), carboxyméthyl cellulose, acide poly(acrylique), polymère biodégradable à base de tyrosine, polyhydroxyalcanoate ou ester de sucre.

12. Procédé selon la revendication 9 ou 10, dans lequel l'adhésif sensible à la pression comporte au moins un élément parmi un adhésif au silicone sensible à la pression, un adhésif au polyuréthanne sensible à la pression, un adhésif acrylique sensible à la pression, un adhésif au cyanoacrylate sensible à la pression, un adhésif à base d'acide poly(lactique-co-glycolique) sensible à la pression, ou un adhésif au polyisobutylène sensible à la pression.

13. Procédé selon l'une quelconque des revendications 9 à 12, comportant en outre la stérilisation de l'accessoire antimicrobien en utilisant au moins une stérilisation parmi une stérilisation par faisceau d'électrons, une stérilisation par faisceau gamma, une stérilisation à l'oxyde d'éthylène, ou un autoclavage.
